# EUROPEAN PATENT APPLICATION

(11) **EP 1 389 461 A1**
(43) Date of publication of application: **18.02.2004**
(21) Application number: 02018357.0
(22) Date of filing: 14.08.2002
(51) Int. Cl.: A61K 31/513, C07D 239/36, C07D 405/06, A61P 31/22

(54) **Pyrimidones with antiviral properties**

(71) Applicant: Axxima Pharmaceuticals Aktiengesellschaft, 82152 Martinsried (DE); 4SC AG, 82152 Martinsried (DE)
(72) Inventor: Missio, Andrea, 81375 München (DE); Herget, Thomas, 81152 Planegg (DE); Aschenbrenner, Andrea, 81241 München (DE); Kramer, Bernd, 52080 Aachen (DE); Leban, Johann, 82110 Germering (DE); Wolf, Kristina, 81373 München (DE)
(74) Representative: Leidescher, Thomas, Dr.

(57) **Abstract**

The present invention relates to vinylpyrimidine-2-one derivatives of the general formula (I): 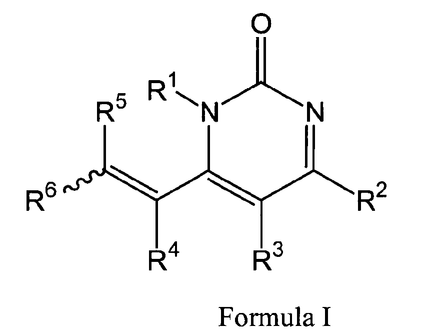 Furthermore the present invention relates to vinylpyrimidine-2-one derivatives and of the general formula (Ia): 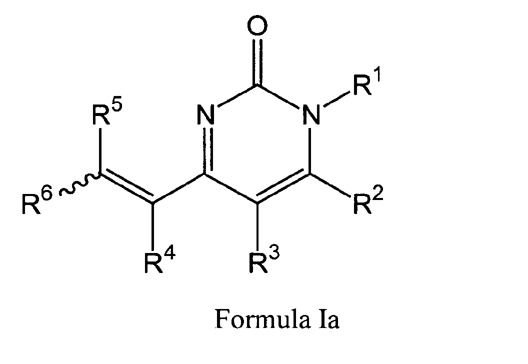 wherein R¹ to R⁶ in formula (I) and in formula (Ia) represent independently of each other a variety of different substituents comprising alkyl, aryl, aralkyl, alkylaryl, heteroaryl groups and monofunctional moieties. The compounds are used as antivirals, especially against herpes and HCMV (Human Cytomegalovirus).

## Description

The present invention relates to vinylpyrimidine-2-one derivatives, the use of the vinylpyrimidine-2-one derivatives as pharmaceutically active agents, especially for the prophylaxis and/or treatment of infectious diseases including opportunistic infections, cell proliferation disorders, cardiovascular disorders, immunological diseases, inflammatory diseases, neuroimmunological diseases, autoimmune diseases, as well as compositions containing at least one vinylpyrimidine-2-one derivative and/or pharmaceutically acceptable salt thereof, and methods for preventing and/or treating such diseases. Furthermore, a process for preparing said vinylpyrimidine-2-one derivatives is disclosed using the corresponding diketones as starting materials.

### Background of the invention

Human Cytomegalovirus (HCMV) is a highly specific β-herpesvirus. Primary infection of healthy children and adults is usually asymptomatic, with a minority of cases developing a mononucleose-like syndrome. In contrast, congenital infection (U.S. 0.2%-2.2% per live birth; aprox. 40,000 per year) leads to several neurological defects in 10-15% of infected neonates. Immunocompromised patients represent another host group facing serious disease complications caused by HCMV infection or reactivation of a persistent infection. Up to 40% of the AIDS patients, for example, develop retinitis, pneumonitis, gastroenteritis or disseminated HCMV disease. In addition, allograft recipients (20,000 allograft transplantations per year in the U.S.) are often infected (or superinfected) by virus from the transplanted organ.

Clinical symptoms in the posttransplant period include prolonged fever, leukopenia, thrombocytopenia, atypical lymphocytosis, elevated hepatic transaminases and decreased graft survival. In bone marrow transplantations, HCMV infection is associated with high mortality rates (80-90% for untreated HCMV pneumonia).

Current approaches to develop therapeutics against Cytomegalovirus (CMV) have focused on antiviral agents *per se;* for example viral polymerase inhibitors. In fact, high mortality rates have been dramatically reduced by new antiviral agents. Current CMV therapeutics possess severe drawbacks, however. For example, Fomivirsen (Vitravene, formerly ISIS 2922) is typically administered by injection directly into the eye every 2 or 4 weeks. Ganciclovir is available for intravenous (Cytovene) or oral administration, and as an implant in the case of retinitis; unfortunately, toxic complications including leukopenia and thrombocytopenia frequently develop. Foscarnet (Foscavir; phosphonoformic acid), another antiviral agent, exhibits considerable renal toxicities and is only available in intravenous form (which is also true for Cidofovir (Vistide), another CMV therapeutic). In addition, CMV replication resumes soon after Ganciclovir and Foscarnet treatment is halted. Finally, Ganciclovir- and Foscarnet-resistant strains of CMV are emerging.

Although treatment of HCMV-induced disease has been improved with these inhibitors of the viral polymerase and preemptive or early antiviral therapy in transplant patients, there is a need in the art for a new class of HCMV therapeutics with better oral bioavailability and reduced toxic effects. This is especially true in the treatment of retinitis in AIDS patients, where CMV infection must be controlled for long periods of time.

Recent research has revealed how cells communicate with each other to coordinate the growth and maintenance of the multitude of tissues within the human body. A key element of this communication network is the transmission of a signal from the exterior of a cell to its nucleus, which results in the activation or suppression of specific genes. This process is called signal transduction.

An integral part of signal transduction is the interaction of cytokines, their receptors, and intracellular signal transduction molecules. Cytokines serve as messengers that bind to receptors on the surface of a target cell. As a result of the binding, the receptors activate a cascade of downstream signaling molecules, thereby transmitting the message from the exterior of the cell to its nucleus. Signal transduction to the nucleus modulates specific gene expression (i.e., transcription and translation), which results in either the upregulation or downregulation of specific proteins that carry out a particular biological function.

Viral infection disrupts normal signal transduction, which leads to cellular malfunctioning resulting in a disease state. Specifically, interference of HCMV with relevant human primary cells is necessary for the virus to create an environment that allows it to grow and replicate, and in turn cause disease in the infected individual. Current research efforts have failed to elucidate all the specific intracellular signal pathways affected by HCMV infection, however. Discovery of the signal transduction pathways and specific intracellular signal transduction molecules affected by CMV infection would represent a tremendous advance in the understanding of the induction and progression of CMV infection processes and provide new avenues for the development of a novel class of effective therapeutics for the treatment of CMV.

It is object of the present invention to provide compounds which can be used as pharmaceutically active agents, especially for prophylaxis and/or treatment of infectious diseases, cell proliferation disorders, cardiovascular disorders, immunological diseases, inflammatory diseases, neuroimmunological diseases, autoimmune diseases, and pharmaceutical compositions comprising said compounds and/or pharmaceutically acceptable salts thereof as pharmaceutically active ingredients.

The object of the present invention is solved by the teaching of the independent claims. Further advantageous features, aspects and details of the invention are evident from the dependent claims, the description, the figures, and the examples of the present application.

The present invention is also directed to novel compounds of the general formula (I) and pharmaceutically acceptable salts thereof: 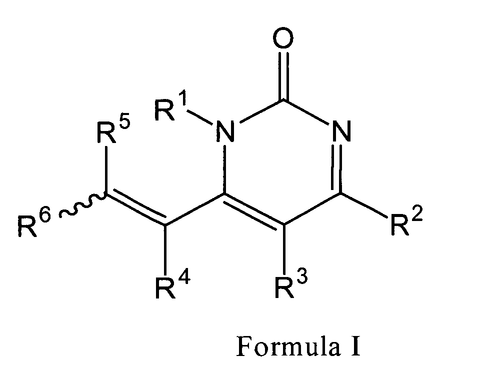 wherein:
R¹ represents C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷, -SO₂R⁷; -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰, R^{10'}, R^{10"} represents F, Cl, Br or l;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
with the proviso that 5-Chloro-1,2,3,6-tetrahydro-1-methyl-2-oxo-6-(2-oxopropyl)-4-pyrimidine carboxylic acid methylester is excluded.

The C₁-C₆-alkyl, C₃-C₈-cycloalkyl, C₅-C₁₅-aryl, and heteroaryl group can optionally be substituted by one or more substituents R⁸.

R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;

R⁹ represents hydrogen, C₁-C₆-hydroxyalkyl, C₁-C₆-alkyl, C₁-C₆-aminoalkyl, C₅-C₁₅-aryl or heteroaryl.

A heteroaryl group represents a 5- or 6-membered heterocyclic group which contains at least one heteroatom like O, N, or S. This heterocyclic group can be fused to another ring. For example, this group can be selected from an oxazole-2-yl, oxazole-4-yl, oxazole-5-yl, thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazole-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo[b]furanyl, benzo[b]thiophenyl, benzimidazolyl, benzothiazolyl, quinazolinyl, quinoxazolinyl, preferably isoxazole-3-yl, isoxazole-4-yl, isoxazole-5-yl, quinolinyl, tetrahydroquinolinyl, isoquinolinyl, tetrahydroisoquinolinyl group. This heterocyclic group can optionally be substituted by one or more substituents R⁸, where R⁸ is as defined above.

The C₁-C₆-alkyl, C₁-C₆-alkenyl, C₁-C₆-alkinyl, and C₃-C₈-cycloalkyl residue may be selected from the group comprising -CH₃, -C₂H₅, -CH=CH₂, -C≡CH, -C₃H₇, -cyclo-C₃H₅, -CH(CH₃)₂, -CH₂-CH=CH₂, -C(CH₃)=CH₂, -CH=CH-CH₃, -C≡C-CH₃, -CH₂-C≡CH, -C₄H₉, -cyclo-C₄H₇, -CH₂-CH(CH₃)₂, -CH(CH₃)-C₂H₅, -C(CH₃)₃, -C₅H₁₁, -cyclo-C₅H₉, -C₆H₁₃, -cyclo-C₆H₁₁, -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇, -C₂H₄-C(R¹⁰)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅, -cyclo-C₇H₁₃, -cyclo-C₈H₁₅ and R¹⁰, R^{10'} and R^{10"} represent independently of each other -H, -F, -Cl, -Br, -I.

The C₅-C₁₅-aryl represents -Ph, -CH₂Ph, -C₂H₄Ph, -CH=CH-Ph, -C≡C-Ph, -o-C₆H₄-R², -m-C₆H₄-R², -p-C₆H₄-R², -o-CH₂-C₆H₄-R², -m-CH₂-C₆H₄-R², -p-CH₂-C₆H₄-R², wherein R² is as defined above.

Furthermore the present invention is directed to novel compounds of the general formula (la) and pharmaceutically acceptable salts thereof: 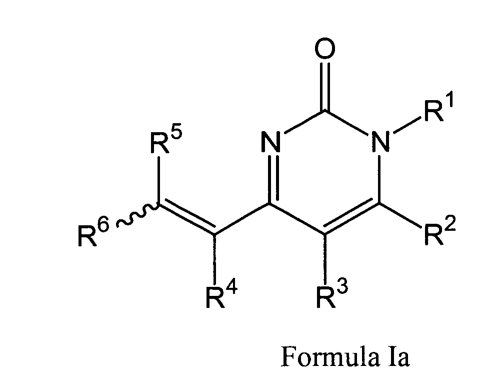 wherein:
R¹ represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₅-C₁₅-aryl or heteroaryl;
with the proviso that the following compounds are excluded:
4-(2,2-diphenylethenyl)-6-methyl-1*H*-pyrimidine-2-one; 4-methyl-6-(2-oxo-2-phenyl-ethyl)-5-phenyl-1*H*-pyrimidine-2-one; 1,2-dihydro-4-methyl-2-oxo-6-(2-phenyl-ethenyl)-ethylester-5-pyrimdine carboxylic acid; 2,3-dihydro-4-[2-(4-methoxy-phenyl)ethenyl]-1,3,6-trimethyl-2-oxo-pyridinium; 2,3-dihydro-4-[2-(4-methoxy-phenyl)ethenyl]-1,3,6-trimethyl-2-oxo-pyridinium perchlorate; 4-phenyl-6-(2-oxo-2-phenylethyl)-1*H*-pyrimidine-2-one; 4-(2,2-diphenylethenyl)-6-phenyl-1*H*-pyrimidine-2-one; 6-phenyl-4-styryl-1*H*-pyrimidine-2-one; 4-(1*H*-indol-2-yl)-6-[(1 E)-2-(2-nitro-phenyl)ethenyl]-1*H*-pyrimidine-2-one; 4-[2-[4-[(2-cyanoethyl) methylamino]-phenyl]-ethenyl]-2,3-dihydro-1,3-dimethyl-2-oxo-pyrimidinium; (*E*)-5-chloro-4-(2-phenyl-ethenyl)-1-(phenylmethyl)-1*H*-pyrimidine-2-one; (*E*)-5-chloro-4-(2-phenylethenyl)-1*H*-pyrimidine-2-one; 4-[2-(4-bromophenyl)-2-oxoethylidene]-1-cyclohexyl-3,4-dihydro-1*H*-pyrimidine-2-one; (Z)-3,4-dihydro-4-(2-oxo-2-phenylethylidene)-1-(phenylmethyl)-1*H*-pyrimidine-2-one; (Z)-5-chloro-3,4-dihydro-1-methyl-4-(2-oxo-2-phenylethylidene)-1*H*-pyrimidine-2-one; (Z)-5-chloro-3,4-dihydro-4-(2-oxo-2-phenyl-ethylidene)-1-phenylmethyl-1*H*-pyrimidine-2-one; 4-[2-[4-(dimethylamino)phenyl]-ethenyl]-2,3-dihydro-2-oxo-1,3-diphenyl-pyrimidinium perchlorate; 4-[2-[4-(di-methylamino)phenyl]ethenyl]-2,3-dihydro-2-oxo-1,3-diphenyl-pyrimidinium; 4-[2-(4-bromophenyl)-2-oxoethylidene]-3,4-dihydro-3-methyl-1*H*-pyrimidine-2-one; 4-[2-(4-chlorophenyl)ethenyl]-1*H*-pyrimidine-2-one; 4-[2-(4-bromophenyl)-2-oxoethylidene]-3,4-dihydro-1-methyl-1*H*-pyrimidine-2-one; 3,4-dihydro-4-[2-(4-methoxyphenyl)-2-oxoethylidene]-1*H*-pyrimidine-2-one; 3,4-dihydro-4-(2-oxo-2-phenylethylidene)-1*H*-pyrimidine-2-one; 4-[2-(4-bromophenyl)-2-oxoethylidene]-3,4-dihydro-1*H*-pyrimidine-2-one; 3,4-dihydro-4-(2-oxo-2-phenylethylidene)-1-β-D-ribofuranosyl-1*H*-pyrimidine-2-one; 4-(2-hydroxy-2-phenylethenyl)-1-β-D-ribofuranosyl-1*H*-pyrimidine-2-one; 4-(2-hydroxy-2-phenylethenyl)-1-(2 ,3, 5-tri-O-benzoyl-,β-D-ribofuranosyl)-1*H*-pyrimidine-2-one; 2,3-dihydro-1,3-dimethyl-4-[2-[4-methyl-2-(methylthio)-5-thiazolyl]ethenyl]-2-oxo-pyrimidinium; 2,3-dihydro-1,3-dimethyl-4-[2-[4-methyl-2-(methylthio)-5-thiazolyl]ethenyl]-2-oxo-pyrimidinium perchlorate; 2,3-dihydro-1,3-dimethyl-4-[2-[2-(methylphenylamino)-4-phenyl-5-thiazolyl]ethenyl]-2-oxo-pyrimidinium; 4-[2-[2-(4-chlorophenyl)-1-methyl-1*H*-indol-3-yl]ethenyl]-2,3-dihydro-1,3-dimethyl-2-oxo-pyrimidinium; 2,3-dihydro-1,3-dimethyl-4-[2-(1-methyl-2-phenyl-1*H*-indol-3-yl)ethenyl]-2-oxo-pyrimidinium; 2,3-dihydro-1,3-dimethyl-4-[2-[4-methyl-2-(methylphenylamino)-5-thiazolyl]ethenyl]-2-oxo-pyrimidinium; 4-[2-[2-(dimethylamino)-4-phenyl-5-thiazolyl]ethenyl]-2,3-dihydro-1,3-dimethyl-2-oxo-pyrimidinium; 2,3-dihydro-1,3-dimethyl-4-[2-[2-(methylthio)-4-phenyl-5-thiazolyl]-ethenyl]-2-oxo-pyrimidinium; 4-[2-[2-(dimethylamino)-5-thiazolyl]ethenyl]-2,3-di-hydro-1,3-dimethyl-2-oxo-pyrimidinium; 4-[2-[4-(dimethylamino)phenyl]ethenyl]-2,3-dihydro-1,3-dimethyl-2-oxo-pyrimidinium; 2,3-dihydro-4-[2-(4-methoxyphenyl)ethenyl]-1,3-dimethyl-2-oxo-pyrimidinium; 4-[2-[2-(dimethylamino)-5-thiazolyl]ethenyl]-2,3-dihydro-1,3-dimethyl-2-oxo-pyrimidinium perchlorate; 4-[(3-ethyl-4-oxo-2-thioxo-5-thiazolidinylidene)ethylidene]-3,4-dihydro-1,3-dimethyl-1*H*-pyrimidine-2-one; 4-[2-[2-(4-chlorophenyl)-1 -methyl-1*H*-indol-3-yl]ethenyl]-2,3-dihydro-1,3-dimethyl-2-oxo-pyrimidinium perchlorate; 2,3-dihydro-1,3-dimethyl-4-[2-(1-methyl-2-phenyl-1*H*-indol-3-yl)ethenyl]-2-oxo-pyrimidinium perchlorate; 2,3-dihydro-1,3-dimethyl-4-[2-[4-methyl-2-(methylphenylamino)-5-thiazolyl]-ethenyl]-2-oxo-pyrimidinium perchlorate; 2,3-dihydro-1,3-dimethyl-4-[2-[2-(methylphenylamino)-4-phenyl-5-thiazolyl]ethenyl]-2-oxo-pyrimidinium perchlorate; 4-[2-[2-(dimethylamino)-4-phenyl-5-thiazolyl]ethenyl]-2,3-dihydro-1,3-dimethyl-2-oxo-pyrimidinium perchlorate; 2,3-dihydro-1,3-dimethyl-4-[2-[2-(methylthio)-4-phenyl-5-thiazolyl]ethenyl]-2-oxo-pyrimidinium perchlorate; 4-[2-[4-(dimethylamino)-phenyl]-ethenyl]-2,3-dihydro-1,3-dimethyl-2-oxo-pyrimidinium perchlorate; 2,3-dihydro-4-[2-(4-methoxyphenyl)ethenyl]-1,3-dimethyl-2-oxo-pyrimidinium perchlorate; 4-(p-chlorophenacylidene)-3,4-dihydro-1 ,3-dimethyl-1*H*-pyrimidine-2-one; 3,4-dihydro-1,3-dimethyl-4-(p-nitrophenacylidene)-1*H*-pyrimidine-2-one; 1,2-dihydro-2-oxo-4-(2-hydrazono-2-phenylethyl)-5-pyrimidinecarboxylic acid hydrazide; 1,2-dihydro-2-oxo-6-styryl-5-pyrimidinecarboxylic acid.

The C₁-C₆-alkyl, C₃-C₈-cycloalkyl, C₅-C₁₅-aryl, and heteroaryl group can optionally be substituted by one or more substituents R⁸.

R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂,-NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;

R⁹ represents hydrogen, C₁-C₆hydroxyalkyl, C₁-C₆-alkyl, C₁-C₆-aminoalkyl, C₅-C₁₅-aryl or heteroaryl.

A heteroaryl group represents a 5- or 6-membered heterocyclic group which contains at least one heteroatom like O, N, or S. This heterocyclic group can be fused to another ring. For example, this group can be selected from an oxazole-2-yl, oxazole-4-yl, oxazole-5-yl, thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1 ,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazole-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo[b]furanyl, benzo[b]thiophenyl, benzimidazolyl, benzothiazolyl, quinazolinyl, quinoxazolinyl, preferably isoxazole-3-yl, isoxazole-4-yl, isoxazole-5-yl, quinolinyl, tetrahydroquinolinyl, isoquinolinyl, tetrahydroisoquinolinyl group. This heterocyclic group can optionally be substituted by one or more substituents R⁸, where R⁸ is as defined above.

The C₁-C₆-alkyl, C₁-C₆-alkenyl, C₁-C₆-alkinyl, and C₃-C₈-cycloalkyl residue may be selected from the group comprising -CH₃, -C₂H₅, -CH=CH₂, -C=CH, -C₃H₇, -cyclo-C₃H₅, -CH(CH₃)₂, -CH₂-CH=CH₂, -C(CH₃)=CH₂, -CH=CH-CH₃, -C≡C-CH₃, -CH₂-C≡CH, -C₄H₉, -cyclo-C₄H₇, -CH₂-CH(CH₃)₂, -CH(CH₃)-C₂H₅, -C(CH₃)₃, -C₅H₁₁, -cyclo-C₅H₉, -C₆H₁₃, -cyclo-C₆H₁₁, -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇, -C₂H₄-C(R¹⁰)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅, -cyclo-C₇H₁₃, -cyclo-C₈H₁₅ and R¹⁰, R^{10'} and R^{10"} represent independently of each other -H, -F, -Cl, -Br, -I.

The C₅-C₁₅-aryl represents -Ph, -CH₂Ph, -C₂H₄Ph, -CH=CH-Ph, -C≡C-Ph, -o-C₆H₄-R², -m-C₆H₄-R², -p-C₆H₄-R², -o-CH₂-C₆H₄-R², -m-CH₂-C₆H₄-R², -p-CH₂-C₆H₄-R², wherein R² is as defined above.

Furthermore the present invention is directed to novel compounds of the general formula (la) and pharmaceutically acceptable salts thereof: 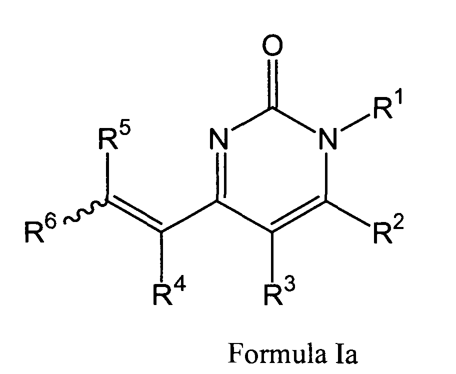 wherein:
R¹ represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃₍R¹⁰)₇ or -C₂H₄-C(R¹⁰)₃;
R¹⁰,R^{10'},R^{10"} represents F, Cl, Br or I;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
with the proviso that the compounds are excluded:
4-(chlorodifluoromethyl )-6-[2-(4-hydroxy-3-methoxyphenyl)ethenyl]-1*H*-pyrimidine-2-one; 4-[2-(1 ,3-benzodioxol-5-yl)ethenyl]-6-chlorodifluoromethyl)-1*H*-pyrimidine-2-one; 4-[2-(3,5-dichloro-2-methoxyphenyl)ethenyl]-6-(trifluoromethyl)-1*H*-pyrimidine-2-one; 4-[2-(6-chloro-1,3-benzodioxol-5-yl)ethenyl]-6-(trifluoromethyl)-1*H*-pyrimidine-2-one; 4-[2-[4-(methylthio)phenyl]ethenyl]-6-(trifluoromethyl)-1*H*-pyrimidine-2-one; 4-(2-(2,3-dichlorophenyl)ethenyl]-6-(trifluoromethyl)-1*H*-pyrimidine-2-one; 4-[2-[5-(2-methoxy-5-nitrophenyl)-2-furanyl]ethenyl]-6-(trifluoromethyl)-1*H*-pyrimidine-2-one; 4-[2-[5-(4-chloro-3-nitrophenyl)-2-furanyl]-ethenyl]-6-(trifluoromethyl)-1*H*-pyrimidine-2-one; 4-[2-[5-(2-chloro-5-nitrophenyl)-2-furanyl]ethenyl]-6-(trifluoromethyl)-1*H*-pyrimidine-2-one; 4-[2-[5-(2-nitrophenyl)-2-furanyl]ethenyl]-6-(trifluoromethyl)-1*H*-pyrimidine-2-one; 4-[2-(3-phenoxy-phenyl)-ethenyl]-6-(trifluoromethyl)-1*H*-pyrimidine-2-one; 4-[2-[4-[(4-fluorophenyl)-methoxy]phenyl]ethenyl]-6-(trifluoromethyl)-1*H*-pyrimidine-2-one; 3-[5-[2-[1,2-di-hydro-2-oxo-6-(trifluoromethyl)-4-pyrimidinyl]ethenyl]-2-furanyl]-benzoic acid methyl ester; 4-[2-[3-ethoxy-4-(phenylmethoxy)phenyl]ethenyl]-6-(trifluoromethyl)-1*H*-pyrimidine-2-one; 4-[2-[4-(1*H*-tetrazol-5-yl)phenyl]ethenyl]-6-(trifluoromethyl)-1*H*-pyrimidine-2-one; 4-[2-[1 ,2-dihydro-2-oxo-6-(trifluoromethyl)-4-pyrimidinyl]-ethenyl]-benzonitrile; 5-chloro-2-[5-[2-[1,2-dihydro-2-oxo-6-(trifluoromethyl)-4-pyrimidinyl]ethenyl]-2-furanyl]-benzoic acid; 4-[2-[5-(3-chloro-4-fluorophenyl)-2-furanyl]ethenyl]-6-(trifluoromethyl)-1*H*-pyrimidine-2-one; 4-[2-(3-phenyl-1*H*-pyrazol-4-yl)ethenyl]-6-(trifluoromethyl)-1*H*-pyrimidine-2-one; 4-[2-[3, 5-d ichloro-2-[(2-fluorophenyl)methoxy]phenyl]ethenyl]-6-(trifluoromethyl)-1*H*-pyrimidine-2-one; 4-[2-(2,4-difluorophenyl)ethenyl]-6-(trifluoromethyl)-1*H*-pyrimidine-2-one; 4-[2-(3-methyl-2-thienyl)ethenyl]-6-(trifluoromethyl)-1*H*-pyrimidine-2-one; 4-[2-[5-(4-nitrophenyl)-2-furanyl]ethenyl]-6-(trifluoromethyl)- 1*H*-pyrimidine-2-one; 4-[2-[2-(benzoyloxy)phenyl]ethenyl]-6-(trifluoromethyl)-1*H*-pyrimidine-2-one; 4-[2-[4-(di-ethylamino)-2-methoxyphenyl]ethenyl]-6-(trifluoromethyl)-1*H*-pyrimidine-2-one; 4-[2-(4-bromo-3-nitrophenyl)ethenyl]-6-(trifluoromethyl)-1*H*-pyrimidine-2-one; 4-[2-[5-(3-chloro-4-methoxyphenyl)-2-furanyl]ethenyl]-6-(trifluoromethyl)-1*H*-pyrimidine-2-one; 4-[2-[1,2-dihydro-2-oxo-6-(trifluoromethyl)-4-pyrimidinyl]ethenyl]-benzoic acid methyl ester; 4-[2-(5-bromo-2-methoxyphenyl)ethenyl]-6-(trifluoromethyl)-1*H*-pyrimidine-2-one; 4-[2-(5-bromo-2-hydroxyphenyl)ethenyl]-6-(trifluoromethyl)-1*H*-pyrimidine-2-one; 4-[2-[5-(3-chloro-4-methylphenyl)-2-furanyl]ethenyl]-6-(trifluoromethyl)-1*H*-pyrimidine-2-one; 4-[2-[4-(1,1-dimethylethyl)phenyl]ethenyl]-6-(tri-fluoromethyl)-1*H*-pyrimidine-2-one; 4-[2-(3-bromo-4-hydroxy-5-methoxy-phenyl)-ethenyl]-6-(trifluoromethyl)-1*H*-pyrimidine-2-one; 4-[2-(2-hydroxy-3,5-diiodo-phenyl)ethenyl]-6-(trifluoromethyl)-1*H*-pyrimidine-2-one; 4-[2-(2-chlorophenyl)-ethenyl]-6-(trifluoromethyl)-1*H*-pyrimidine-2-one; 4-[2-(2,4-dimethoxyphenyl)-ethenyl]-6-(trifluoromethyl)-1*H*-pyrimidine-2-one; 4-[2-(6-bromo-1,3-benzodioxol-5-yl)ethenyl]-6-(trifluoromethyl)-1*H*-pyrimidine-2-one; 4-[2-(5-chloro-2-hydroxyphenyl)ethenyl]-6-(trifluoromethyl)-1*H*-pyrimidine-2-one; 4-[2-(4-bromphenyl)-ethenyl]-6-(trifluoromethyl)-1*H*-pyrimidine-2-one; 4-[2-(3,4-dimethoxyphenyl)-ethenyl]-6-(trifluoromethyl)-1*H*-pyrimidine-2-one; 4-[2-[4-(diethylamino)-2-hydroxyphenyl]ethenyl]-6-(trifluoromethyl)-1*H*-pyrimidine-2-one; 4-[2-(3,5-d ibromo-2-hydroxyphenyl)ethenyl]-6-(trifluoromethyl)-1*H*-pyrimidine-2-one; 5-[2-[1,2-dihydro-2-oxo-6-(trifluoromethyl)-4-pyrimidinyl]ethenyl]-1,3-dihydro-1,3-dimethyl-2*H*-benzimidazole-2-one; 5-[5-[2-[1,2-dihydro-2-oxo-6-(trifluoromethyl)-4-pyrimidinyl]-ethenyl]-2-furanyl]-2-hydroxy-benzoic acid; 4-[5-[2-[1,2-dihydro-2-oxo-6-(trifluoromethyl)-4-pyrimidinyl]ethenyl]-2-furanyl]-benzoic acid; 4-[2-[5-(4-bromophenyl)-2-furanyl]ethenyl]-6-(trifluoromethyl)-1*H*-pyrimidine-2-one; 4-[2-(4-chlorophenyl)-ethenyl]-6-(trifluoromethyl)-1*H*-pyrimidine-2-one; 4-[2-[5-(3-chlorophenyl)-2-furanyl]ethenyl]-6-(trifluoromethyl)-1*H*-pyrimidine-2-one; 4-[2-(3-bromophenyl)-ethenyl]-6-(trifluoromethyl)- 1*H*-pyrimidine-2-one; 4-[2-[4-(1 -pyrrolidinyl)phenyl]-ethenyl]-6-(trifluoromethyl)-1*H*-pyrimidine-2-one; 2-chloro-4-[5-[2-[1,2-dihydro-2-oxo-6-(trifluoromethyl)-4-pyrimidinyl]ethenyl]-2-furanyl]-benzoic acid; 4-[2-[5-(2-chloro-4-nitrophenyl)-2-furanyl]ethenyl]-6-(trifluoromethyl)-1*H*-pyrimidine-2-one; 4-[2-[5-(2-fluorophenyl)-2-furanyl]ethenyl]-6-(trifluoromethyl)-1*H*-pyrimidine-2-one; 4-chloro-2-[5-[2-[1,2-dihydro-2-oxo-6-(trifluoromethyl)-4-pyrimidinyl]ethenyl]-2-furanyl]-benzoic acid; 4-[2-(4-methoxyphenyl)ethenyl]-6-(trifluoromethyl)-1*H*-pyrimidine-2-one; 4-[2-(2-furanyl)ethenyl]-6-(trifluoromethyl)-1*H*-pyrimidine-2-one; 4-[2-[4-(dimethylamino)-3-nitrophenyl]ethenyl]-6-(trifluoromethyl)-1*H*-pyrimidine-2-one; 4-[2-(2-hydroxyphenyl)ethenyl]-6-(trifluoromethyl)-1*H*-pyrimidine-2-one; 4-[2-(4-hydroxyphenyl)ethenyl]-6-(trifluoromethyl)-1*H*-pyrimidine-2-one; 4-[2-[5-(2,5-dimethyl-3-nitrophenyl)-2-furanyl]ethenyl]-6-(trifluoromethyl)-1*H*-pyrimidine-2-one; 4-[2-(3,5-dibromo-2-methoxyphenyl)ethenyl]-6-(trifluoromethyl)-1*H*-pyrimidine-2-one; 4-[2-[5-(2,5-dimethyl-4-nitrophenyl)-2-furanyl]ethenyl]-6-(trifluoromethyl)-1*H*-pyrimidine-2-one; 4-[2-(3-ethoxy-4-hydroxyphenyl)ethenyl]-6-(trifluoromethyl)-1*H*-pyrimidine-2-one; 3-[5-[2-[1,2-dihydro-2-oxo-6-(trifluoromethyl)-4-pyrimidinyl]-ethenyl]-2-furanyl]-benzoic acid; 4-[2-(2,4-dichlorophenyl)ethenyl]-6-(trifluoromethyl)-1*H*-pyrimidine-2-one; 4,4'-(1,4-phenylenedi-2,1-ethenediyl)bis[6-(trifluoromethyl)-1*H*-pyrimidine-2-one; 4-[2-[5-(2,4-dimethyl-5-nitrophenyl)-2-furanyl]-ethenyl]-6-(trifluoromethyl)-1*H*-pyrimid ine-2-one; 4-[2-[5-(2-methoxy-4-nitrophenyl)-2-furanyl]ethenyl]-6-(trifluoromethyl)-1*H*-pyrimidine-2-one; 4-[2-[5-(2,4-dichlorophenyl)-2-furanyl]ethenyl]-6-(trifluoromethyl)-1*H*-pyrimidine-2-one; 4-[2-[3,5-dibromo-2-[(2,4-dichlorophenyl)methoxy]phenyl]ethenyl]-6-(trifluoromethyl)-1*H*-pyrimidine-2-one; 4-[2-[5-(3-nitrophenyl)-2-furanyl]ethenyl]-6-(trifluoromethyl)-1*H*-pyrimidine-2-one; 4-[2-(2,4-dichloro-5-nitrophenyl)ethenyl]-6-(trifluoromethyl)-1*H*-pyrimidine-2-one; 4-[2-[4-(dimethylamino)phenyl]ethenyl]-6-(trifluoromethyl)-1*H*-pyrimidine-2-one; 4-[2-(1 -naphthalenyl)ethenyl]-6-(trifluoromethyl)-1*H*-pyrimidine-2-one; 4-[2-(3-fluorophenyl)ethenyl]-6-(trifluoromethyl)-1*H*-pyrimidine-2-one; 4-(trifluoromethyl)-6-[2-(2,4,5-trimethylphenyl)ethenyl]-1*H*-pyrimidine-2-one; 4-[2-[4-(4-morpholinyl)phenyl]ethenyl]-6-(trifluoromethyl)-1*H*-pyrimidine-2-one; 4-[2-(9-ethyl-9H-carbazol-3-yl)ethenyl]-6-(trifluoromethyl)-1*H*-pyrimidine-2-one; 4-[2-[5-(4-methoxy-2-nitrophenyl)-2-furanyl]ethenyl]-6-(trifluoromethyl)-1*H*-pyrimidine-2-one; [2-[2-[1,2-dihydro-2-oxo-6-(trifluoromethyl)-4-pyrimidinyl]-ethenyl]-6-methoxyphenoxy]-acetic acid ethyl ester; 4-[2-(1,3-benzodioxol-5-yl )ethenyl]-6-(trifluoromethyl)-1*H*-pyrimidine-2-one; 4-[2-[1 -[(2-fluorophenyl)-methyl]-1*H*-indol-3-yl]ethenyl]-6-(trifluoromethyl)-1*H*-pyrimidine-2-one; [4-[2-[1,2-dihydro-2-oxo-6-(trifluoromethyl)-4-pyrimidinyl]ethenyl]-2-methoxyphenoxy]-acetic acid ethyl ester; 4-[2-[5-(5-chloro-2-methoxyphenyl)-2-furanyl]ethenyl]-6-(trifluoromethyl)-1*H*-pyrimidine-2-one; 4-[2-(2,3-dimethoxyphenyl)ethenyl]-6-(trifluoromethyl)-1*H*-pyrimidine-2-one; 8-[4-[2-[1,2-dihydro-2-oxo-6-(trifluoromethyl)-4-pyrimidinyl]ethenyl]-2-methoxyphenoxy]-3, 7-dihydro-1,3,7-trimethyl-1*H*-purine-2,6-dione; 4-[2-[5-(1,3-dihydro-1-oxo-5-isobenzofuranyl)-2-furanyl]ethenyl]-6-(trifluoromethyl)-1*H*-pyrimidine-2-one; 4-[2-[1-[(3,4-dichlorophenyl)methyl]-3-phenyl-1*H*-pyrazol-4-yl]ethenyl]-6-(trifluoromethyl)-1*H*-pyrimidine-2-one; 4-[2-[5-(2,3-dichlorophenyl)-2-furanyl]ethenyl]-6-(trifluoromethyl)-1*H*-pyrimidine-2-one; 4-[2-(4-[(2,4-dichlorophenyl)methoxy]-3-methoxyphenyl]ethenyl]-6-(trifluoromethyl)-1*H*-pyrimidine-2-one; 4-[2-(4-hydroxy-3-methoxyphenyl)ethenyl]-6-(trifluoromethyl)-1*H*-pyrimidine-2-one; 4-[2-(2-chloro-5-nitrophenyl)ethenyl]-6-(trifluoromethyl)-1*H*-pyrimidine-2-one; 4-[2-[5-(4-fluorophenyl)-2-furanyl]ethenyl]-6-(trifluoromethyl)-1*H*-pyrimidine-2-one; 4-[2-[5-(3,5-dichlorophenyl)-2-furanyl]ethenyl]-6-(trifluoromethyl)-1*H*-pyrimidine-2-one; 4-(trifluoromethyl)-6-[2-[5-[3-(trifluoromethyl)phenyl]-2-furanyl]ethenyl]-1*H*-pyrimidine-2-one; 2-chloro-5-[5-[2-[1,2-dihydro-2-oxo-6-(trifluoromethyl)-4-pyrimidinyl]ethenyl]-2-furanyl]-benzoic acid; 4-[2-(7-methoxy-2-oxo-1,3-benzoxathiol-5-yl)ethenyl]-6-(trifluoromethyl)-1*H*-pyrimidine-2-one; 4-[2-(3-nitrophenyl)ethenyl]-6-(trifluoromethyl)-1*H*-pyrimidine-2-one; 4-[2-(4-hydroxy-3,5-dimethoxyphenyl)ethenyl]-6-(trifluoromethyl)-1*H*-pyrimidine-2-one; 4-[2-(3-hydroxyphenyl)ethenyl]-6-(trifluoromethyl)-1*H*-pyrimidine-2-one; 4-(trifluoromethyl)-6-[2-(2,4,5-trimethoxyphenyl)ethenyl]-1*H*-pyrimidine-2-one; 4-[2-(3,5-diiodo-2-methoxyphenyl)ethenyl]-6-(trifluoromethyl)-1*H*-pyrimidine-2-one; 4-[2-(3-ethoxy-4-methoxyphenyl)ethenyl]-6-(trifluoromethyl)-1*H*-pyrimidine-2-one; 4-(trifluoromethyl)-6-[2-(3,4, 5-trimethoxyphenyl)ethenyl]-1*H*-pyrimidine-2-one; 4-[2-(3-bromo-4-methoxyphenyl)ethenyl]-6-(trifluoromethyl)-1*H*-pyrimidine-2-one; 2-[5-[2-[1,2-dihydro-2-oxo-6-(trifluoromethyl)-4-pyrimidinyl]ethenyl]-2-furanyl]-benzoic acid; 4-[2-(6-nitro-1,3-benzodioxol-5-yl )ethenyl]-6-(trifluoromethyl)-1*H*-pyrimidine-2-one; 4-(trifluoromethyl)-6-[2-[2-(trifluoromethyl)phenyl]ethenyl]-1*H*-pyrimidine-2-one; 4-[2-(2-hydroxy-1 -naphthalenyl)ethenyl]-6-(trifluoromethyl)-1*H*-pyrimidine-2-one; 4-[2-[1-(4-nitrophenyl)-1*H*-pyrrol-2-yl]ethenyl]-6-(trifluoromethyl)-1*H*-pyrimidine-2-one.

The C₁-C₆-alkyl, C₃-C₈-cycloalkyl, C₅-C₁₅-aryl, and heteroaryl group can optionally be substituted by one or more substituents R⁸.

R⁸ represents independently of each other hydrogen, -COOR⁹, -CONHR⁹, -F, -Cl, - Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;

R⁹ represents hydrogen, C₁-C₆-hydroxyalkyl, C₁-C₆-alkyl, C₁-C₆-aminoalkyl, C₅-C₁₅-aryl or heteroaryl.

A heteroaryl group represents a 5- or 6-membered heterocyclic group which contains at least one heteroatom like O, N, or S. This heterocyclic group can be fused to another ring. For example, this group can be selected from an oxazole-2-yl, oxazole-4-yl, oxazole-5-yl, thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo[b]furanyl, benzo[b]thiophenyl, benzimidazolyl, benzothiazolyl, quinazolinyl, quinoxazolinyl, preferably isoxazole-3-yl, isoxazole-4-yl, isoxazole-5-yl, quinolinyl, tetrahydroquinolinyl, isoquinolinyl, tetrahydroisoquinolinyl group. This heterocyclic group can optionally be substituted by one or more substituents R⁸, where R⁸ is as defined above.

The C₁-C₆-alkyl, C₁-C₆-alkenyl, C₁-C₆-alkinyl, and C₃-C₈-cycloalkyl residue may be selected from the group comprising -CH₃, -C₂H₅, -CH=CH₂, -C≡CH, -C₃H₇, -cyclo-C₃H₅, -CH(CH₃)₂, -CH₂-CH=CH₂, -C(CH₃)=CH₂, -CH=CH-CH₃, -C≡C-CH₃, -CH₂-C≡CH, -C₄H₉, -cyclo-C₄H₇, -CH₂-CH(CH₃)₂, -CH(CH₃)-C₂H₅, -C(CH₃)₃, -C₅H₁₁, -cyclo-C₅H₉, -C₆H₁₃, -cyclo-C₆H₁₁, -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇, -C₂H₄-C(R¹⁰)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C ≡C-CH₃, -CH₂-C≡C-C₂H₅, -CH₂-C≡C-CH=CH₂, -CH₂-CH=CH-C=CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅, -cyclo-C₇H₁₃, -cyclo-C₈H₁₅ and R¹⁰, R^{10'} and R^{10"} represent independently of each other -H, -F, -Cl, -Br, -I.

The C₅-C₁₅-aryl represents -Ph, -CH₂Ph, -C₂H₄Ph, -CH=CH-Ph, -C≡C-Ph, -o-C₆H₄-R², -m-C₆H₄-R², -p-C₆H₄-R², -o-CH₂-C₆H₄-R², -m-CH₂-C₆H₄-R², -p-CH₂-C₆H₄-R², wherein R² is as defined above.

Most preferred are the following compounds:
4-{2-[4-(2-Chloro-6-fluoro-benzyloxy)-3,5-dimethoxy-phenyl]-vinyl}-6-trifluoromethyl-1H-pyrimidine-2-one; 6-Trifluoromethyl-4-[2-(4-trifluoromethyl-phenyl)-vinyl]-1H-pyrimidine-2-one; 4-[2-(2-Hydroxy-4-methoxy-phenyl)-vinyl]-6-trifluoromethyl-1H-pyrimidine-2-one; 4-[2-(5-Bromo-2-hydroxy-3-methoxy-phenyl)-vinyl]-6-trifluoro-methyl-1H-pyrimidine-2-one; 4-[2-(2,6-Dichloro-phenyl)-vinyl]-6-trifluoromethyl-1H-pyrimidine-2-one; 5-(4-Chloro-phenyl)-2-[2-(2-oxo-6-trifluoromethyl-1,2-dihydro-pyrimidine-4-yl)-vinyl]-furan-3-carboxylic acid ethyl ester; 4-[2-(2-Oxo-6-trifluoromethyl-1,2-dihydro-pyrimidin-4-yl)-vinyl]-benzoic acid; 6-Trifluoromethyl-4-{2-[5-(2-trifluoromethyl-phenyl)-furan-2-yl]-vinyl}-1H-pyrimidine-2-one; 5-{1 -[2-(2-Oxo-6-trifluoromethyl-1,2-dihydro-pyrimidine-4-yl)-vinyl]-naphthalen-2-yloxymethyl}-4,5-dihydro-isoxazole-3-carboxylic acid ethyl ester; 4-[2-(2-Allyloxy-naphthalen-1 -yl)-vinyl]-6-trifluoromethyl-1H-pyrimidine-2-one; 4-[2-(3-Phenyl-benzo[c]isoxazole-5-yl)-vinyl]-6-trifluoromethyl-1H-pyrimidine-2-one; 4-{2-[2-(2-Oxo-6-trifluoromethyl-1 ,2-dihydro-pyrimidine-4-yl)-vinyl]-phenyl}-piperazine-1-carboxylic acid ethyl ester; 6-Trifluoromethyl-4-(2-{2-[4-(5-trifluoromethyl-pyridine-2-yl)-piperazin-1 -yl]-phenyl}-vinyl)-1H-pyrimidin-2-one; 4-{2-[2-(4-Benzyl-piperazine-1-yl)-phenyl]-vinyl}-6-tri-fluoromethyl-1H-pyrimidine-2-one; 2-Chloro-5-{5-[2-(2-oxo-6-trifluoromethyl-1,2-dihydro-pyrimidine-4-yl)-vinyl]-furan-2-yl}-benzoic acid; 4-{2-[5-(3-Methyl-4-nitro-phenyl)-furan-2-yl]-vinyl}-6-trifluoromethyl-1H-pyrimidine-2-one; 6-Methyl-4-[2-(4-tri-fluoromethyl-phenyl)-vinyl]-1H-pyrimidine-2-one; 4,6-Bis-[2-(5-bromo-2-hydroxy-3-methoxy-phenyl)-vinyl]-pyrimidine-2-ol; 4-{2-[5-(2-Chloro-phenyl)-furan-2-yl]-vinyl}-6-trifluoromethyl-1H-pyrimidine-2-one; 4-{2-[5-(4-Chloro-phenyl)-furan-2-yl]-vinyl}-6-trifluoromethyl-1H-pyrimidine-2-one; 4-[2-(5-Hydroxymethyl-furan-2-yl)-vinyl]-6-tri-fluoromethyl-1H-pyrimidine-2-one; 4,6-Bis-[2-(2-trifluoromethyl-phenyl)-vinyl]-pyrimidine-2-ol; 4-[2-(2,6-Dichloro-phenyl)-vinyl]-6-methyl-pyrimidine-2-ol; 4-[2-(5-Methyl-thiophen-2-yl)-vinyl]-6-trifluoromethyl-1H-pyrimidine-2-one; 4-(2-Thiophen-2-yl-vinyl)-6-trifluoromethyl-1H-pyrimidine-2-one; 4-[2-(4-Bromo-5-tert-butyl-thiophen-2-yl)-vinyl]-6-trifluoromethyl-1H-pyrimidine-2-one; 4-{2-[3-(2,4-Dichloro-benzyloxy)-naphthalen-2-yl]-vinyl}-6-trifluoromethyl-1H-pyrimidine-2-one; 4-[2-(2-Hydroxynaphthalen-1-yl)-vinyl]-6-methyl-pyrimidine-2-ol; 4-[2-(3,5-Dibromo-2-hydroxyphenyl)-vinyl]-6-methyl-pyrimidine-2-ol; 4-{2-[5-(2-Chloro-5-trifluoromethyl-phenyl)-furan-2-yl]-vinyl}-6-trifluoromethyl-1H-pyrimidine-2-one; 4-[2-(5-Bromo-furan-2-yl)-vinyl]-6-trifluoromethyl-1H-pyrimidine-2-one; 4-(2-Benzofuran-2-yl-vinyl)-6-trifluoromethyl-1H-pyrimidine-2-one; 4-[2-(5-Bromo-thiophen-2-yl)-vinyl]-6-trifluoromethyl-1H-pyrimidine-2-one; 2-[2-(2-Oxo-6-trifluoromethyl-1,2-dihydro-pyrimidine-4-yl)-vinyl]-thiophene-3-carboxylic acid; 4-[2-(4-Trifluoromethyl-phenyl)-vinyl]-pyrimidine-2-ol; 4-[2-(2-Trifluoromethyl-phenyl)-vinyl]-pyrimidine-2-ol; 4-[2-(3,5-Dibromo-2-hydroxy-phenyl)-vinyl]-pyrimidine-2-ol; 4-[2-(2,6-Dichloro-phenyl)-vinyl]-pyrimidine-2-ol; 4-[2-(2-Hydroxy-naphthalen-1 -yl)-vinyl]-pyrimidine-2-ol; 4-[2-(5-Bromo-2-hydroxy-3-methoxy-phenyl)-vinyl]-6-methyl-pyrimidine-2-ol; 4-[2-(2-Hydroxy-naphthalen-1-yl)-1-methyl-vinyl]-6-trifluoromethyl-1H-pyrimidine-2-one; 4-[2-(2-Hydroxynaphthalen-1-yl)-vinyl]-6-phenyl-1H-pyrimidine-2-one; 4-[1-Methyl-2-(4-trifluoromethyl-phenyl)-vinyl]-6-trifluoromethyl-1H-pyrimidine-2-one; 6-(Chlorodifluoromethyl)-4-[2-(4-trifluoromethyl-phenyl)-vinyl]-1H-pyrimidine-2-one; 6-Phenyl-4-[2-(4-trifluoromethyl-phenyl)-vinyl]-1H-pyrimidine-2-one; 6-(Chlorodifluoromethyl)-4-[2-(3,5-dibromo-2-hydroxy-phenyl)-vinyl]-1H-pyrimidine-2-one; 4-[2-(3, 5-Dibromo-2-hydroxy-phenyl)-vinyl]-6-phenyl-1H-pyrimidine-2-one; 4-[2-(2,6-Dichloro-phenyl)-1 -methyl-vinyl]-6-trifluoromethyl-1H-pyrimidine-2-one; 6-(Chloro-difluoro-methyl)-4-[2-(2,6-dichloro-phenyl)-vinyl]-1H-pyrimidine-2-one; 4-[2-(2,6-Dichloro-phenyl)-vinyl]-6-phenyl-1H-pyrimidine-2-one; 4-[2-(3,5-Dibromo-2-hydroxy-phenyl)-1 -methyl-vinyl]-6-trifluoromethyl-1H-pyrimidine-2-one; 4-[1-Methyl-2-(2-trifluoro-methylphenyl)-vinyl]-6-trifluoromethyl-1H-pyrimidine-2-one; 6-Phenyl-4-[2-(2-tri-fluoromethylphenyl)-vinyl]-1H-pyrimidine-2-one.

For the first time the present invention provides compounds of the following general formula (I) and pharmaceutically acceptable salts thereof, 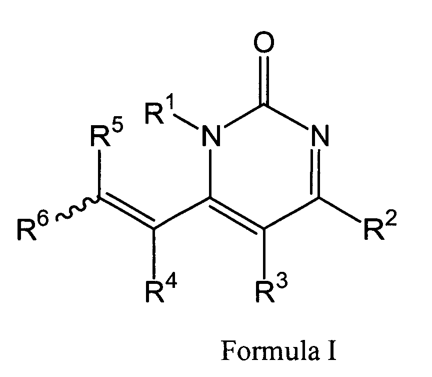 wherein:
R¹ represents C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷, -SO₂R⁷, ₋C(R¹⁰)₃, -CR¹⁰(R¹⁰')₂, -CR¹⁰(R^{10'})R¹⁰", -C₂(R¹⁰)_{5,} -CH₂-C(R¹⁰)_{3,} -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰, R^{10'}, R^{10"} represents F, Cl, Br or l;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
for the use as pharmaceutically active agent.

The C₁-C₆-alkyl, C₃-C₈-cycloalkyl, C₅-C₁₅-aryl, and heteroaryl group can optionally be substituted by one or more substituents R⁸.

R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl; R⁹ represents hydrogen, C₁-C₆-hydroxyalkyl, C₁-C₆-alkyl, C₁-C₆-aminoalkyl, C₅-C₁₅-aryl or heteroaryl.

A heteroaryl group represents a 5- or 6-membered heterocyclic group which contains at least one heteroatom like O, N, or S. This heterocyclic group can be fused to another ring. For example, this group can be selected from an oxazole-2-yl, oxazole-4-yl, oxazole-5-yl, thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo[b]furanyl, benzo[b]thiophenyl, benzimidazolyl, benzothiazolyl, quinazolinyl, quinoxazolinyl, preferably isoxazole-3-yl, isoxazole-4-yl, isoxazole-5-yl, quinolinyl, tetrahydroquinolinyl, isoquinolinyl, tetrahydroisoquinolinyl group. This heterocyclic group can optionally be substituted by one or more substituents R⁸, where R⁸ is as defined above.

The C₁-C₆-alkyl, C₁-C₆-alkenyl, C₁-C₆-alkinyl, and C₃-C₈-cycloalkyl residue may be selected from the group comprising -CH₃, -C₂H₅, -CH=CH₂, -C≡CH, -C₃H₇, -cyclo-C₃H₅, -CH(CH₃)₂, -CH₂-CH=CH₂, -C(CH₃)=CH₂, -CH=CH-CH₃, -C≡C-CH₃, -CH₂-C≡CH, -C₄H₉, -cyclo-C₄H₇, -CH₂-CH(CH₃)₂, -CH(CH₃)-C₂H₅, -C(CH₃)₃, -C₅H₁₁, -cyclo-C₅H₉, -C₆H₁₃, -cyclo-C₆H₁₁, -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇, -C₂H₄-C(R¹⁰)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡-CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅, -cyclo-C₇H₁₃, -cyclo-C₈H₁₅ and R¹⁰, R^{10'} and R^{10"} represent independently of each other -H, -F, -Cl, -Br, -I.

The C₅-C₁₅-aryl represents -Ph, -CH₂Ph, -C₂H₄Ph, -CH=CH-Ph, -C≡C-Ph, -o-C₆H₄-R², -m-C₆H₄-R², -p-C₆H₄-R², -o-CH₂-C₆H₄-R², -m-CH₂-C₆H₄-R², -p-CH₂-C₆H₄-R², wherein R² is as defined above.

Beside these residues the C₅-C₁₅-aryl, C₃-C₈-cycloalkyl and heteroaryl represent independently of each other 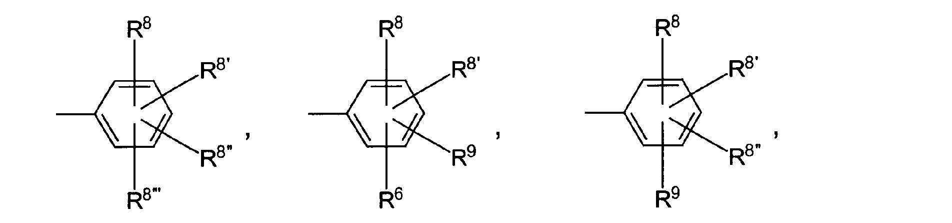 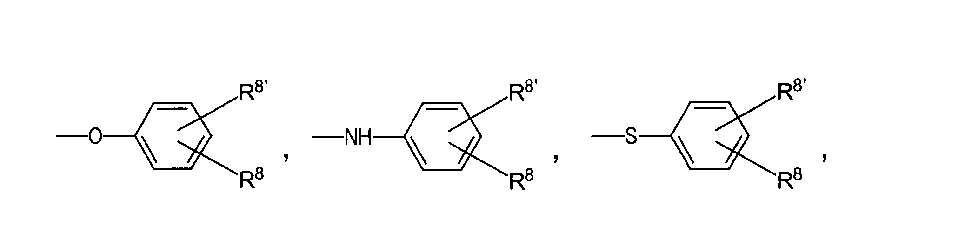 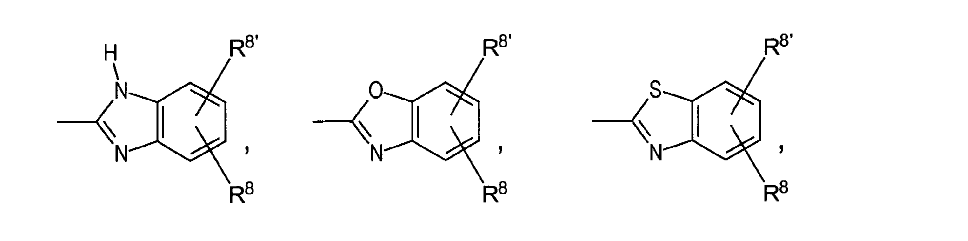 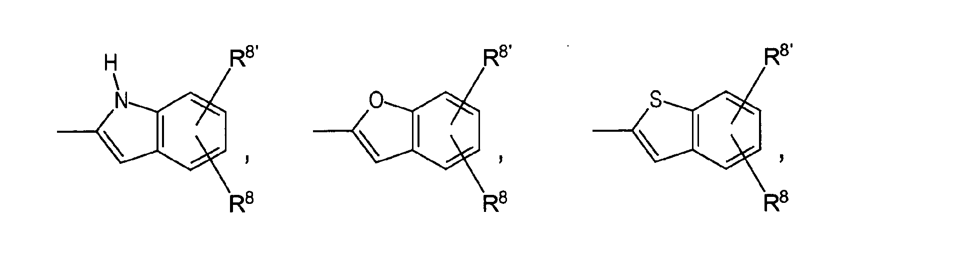 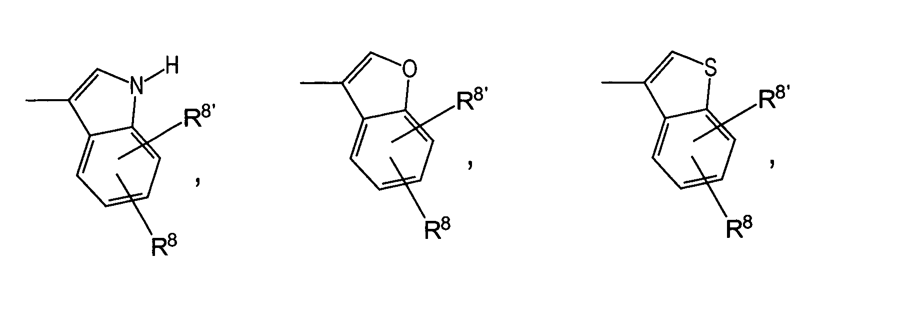 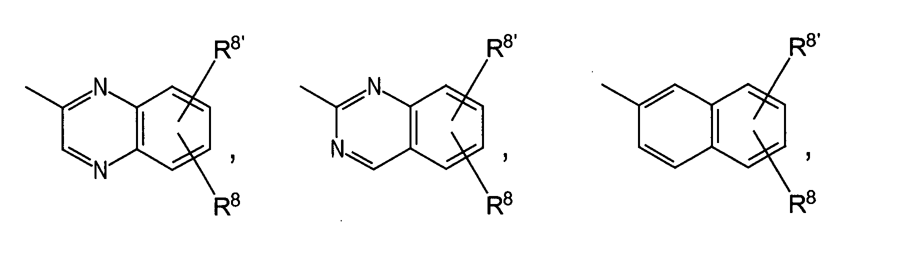 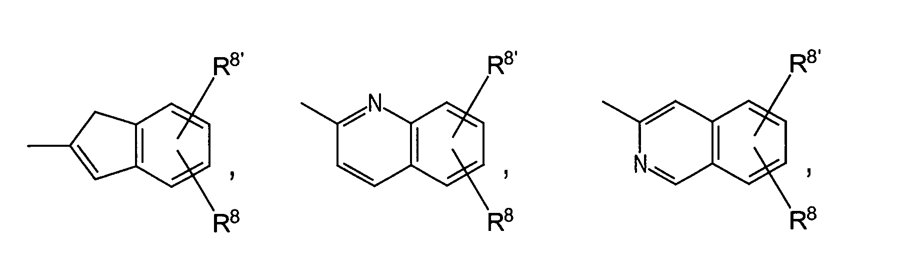 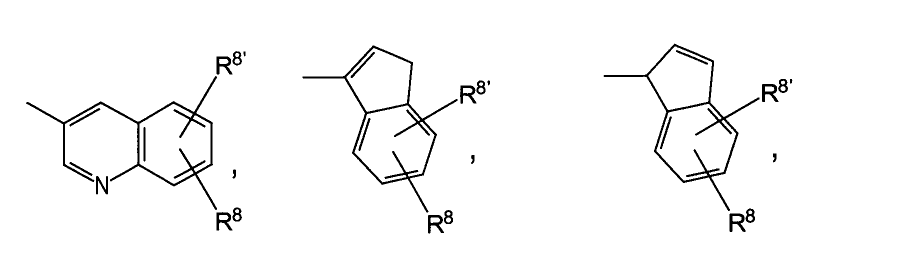 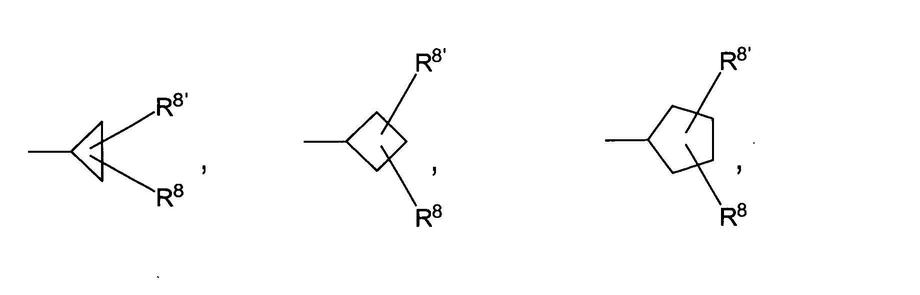 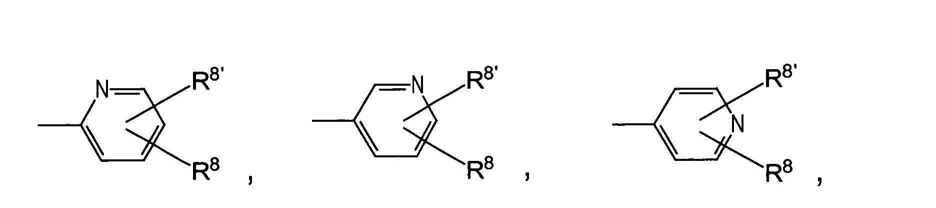 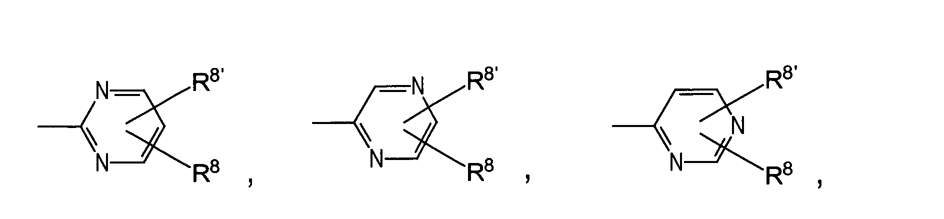 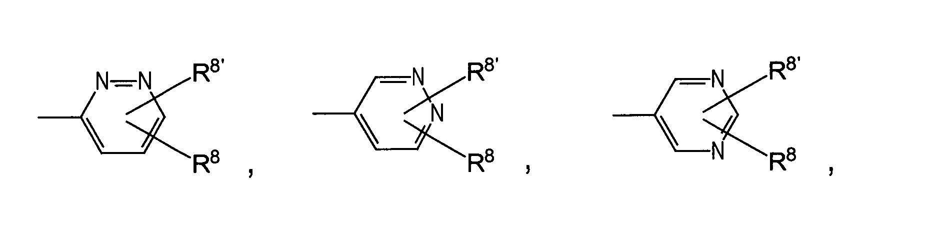 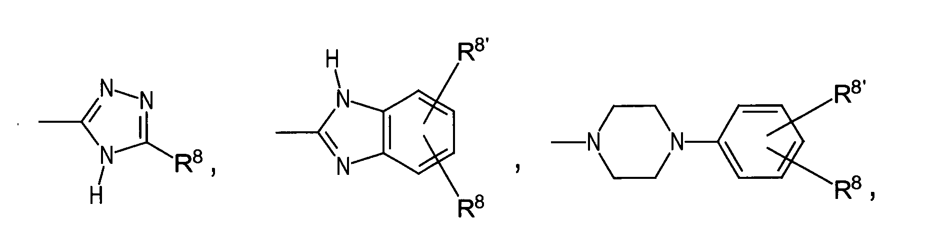 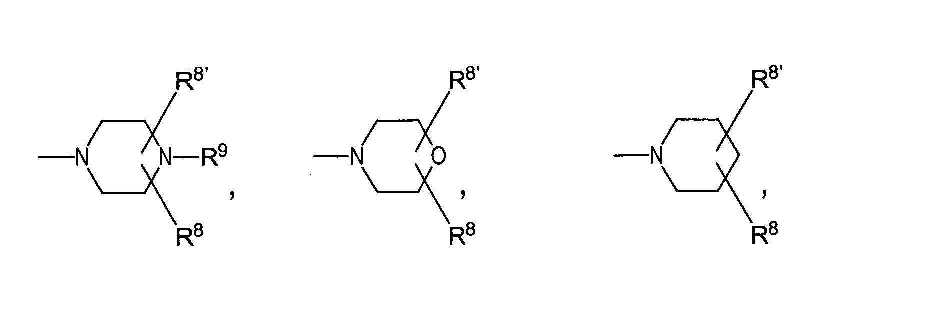 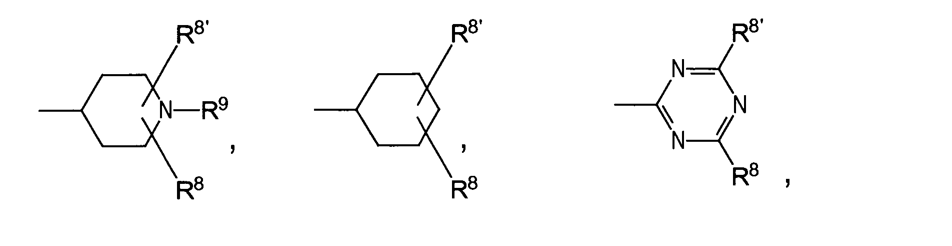 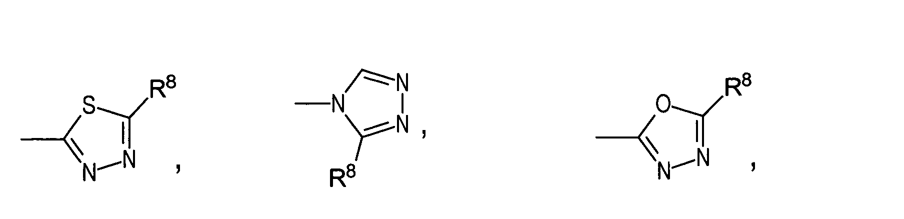 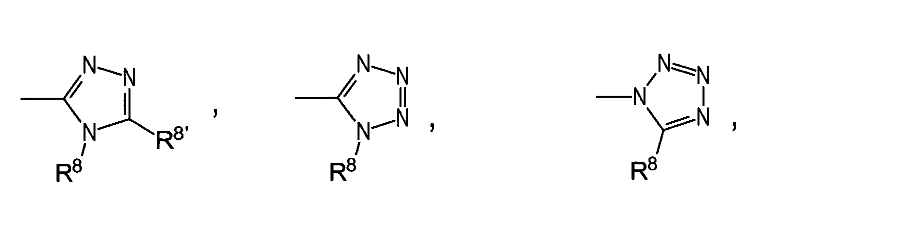 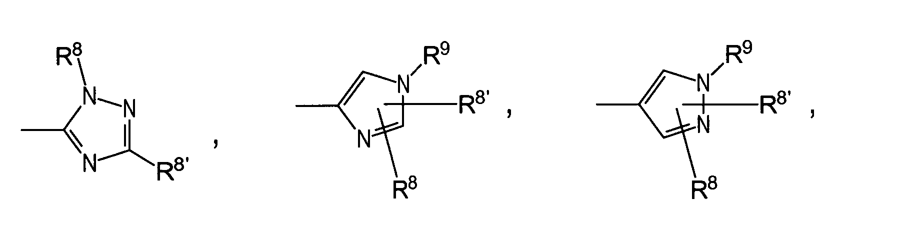 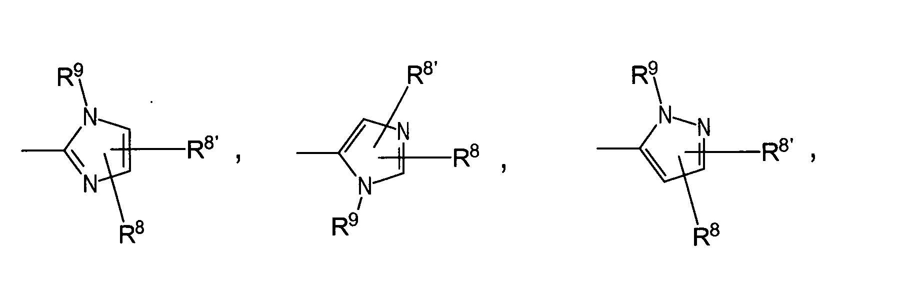 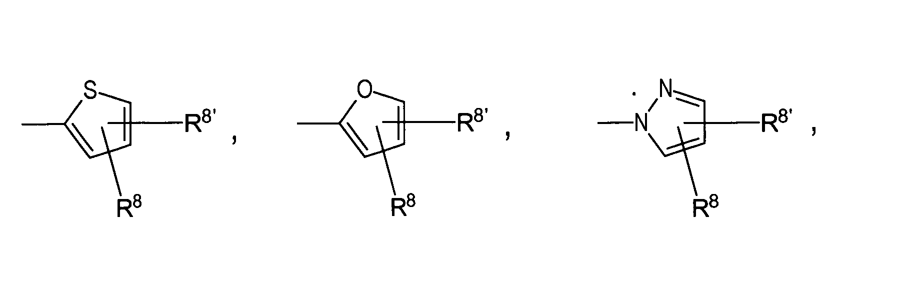 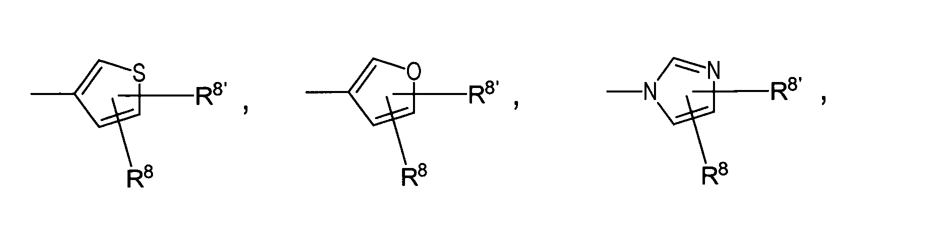 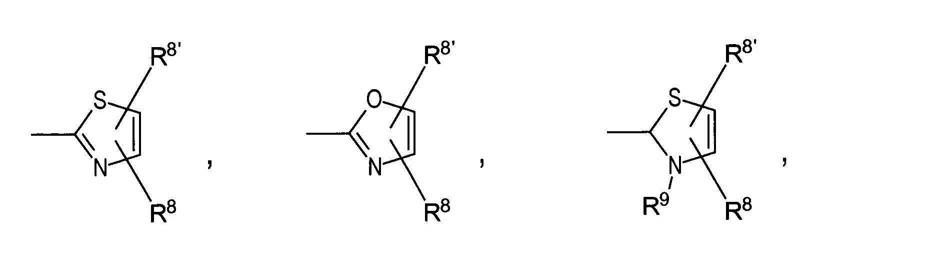 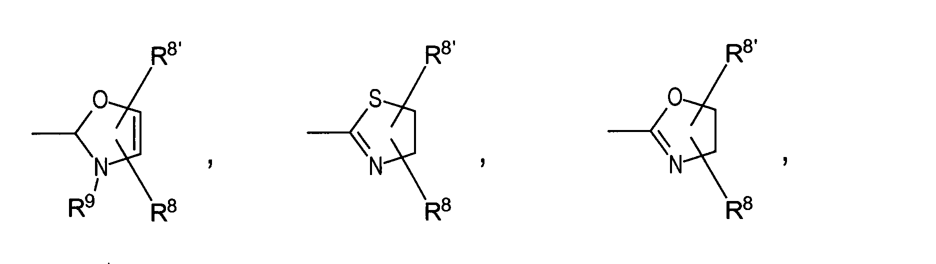 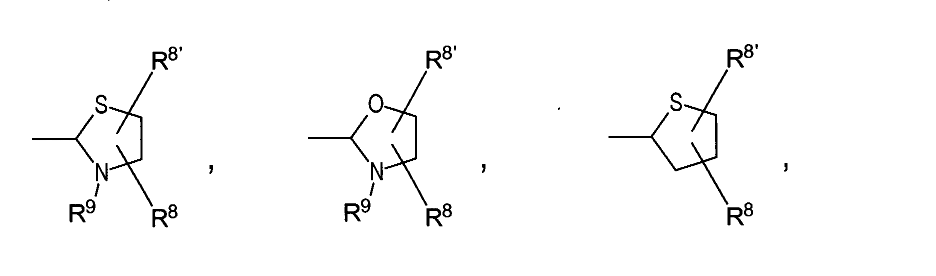 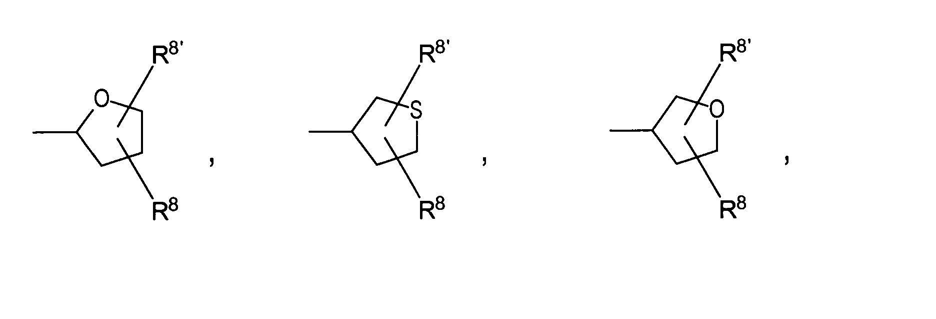 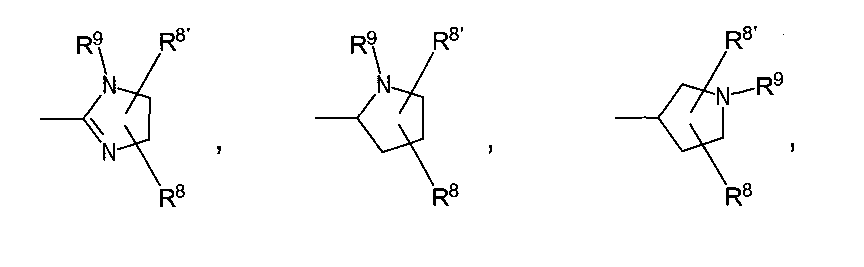 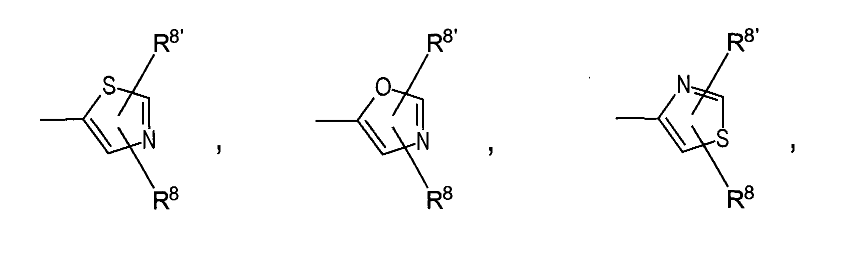 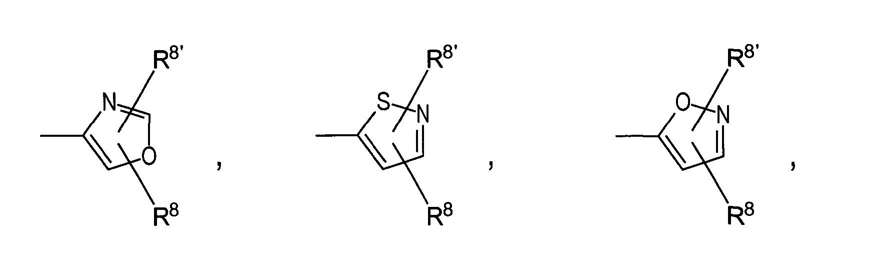 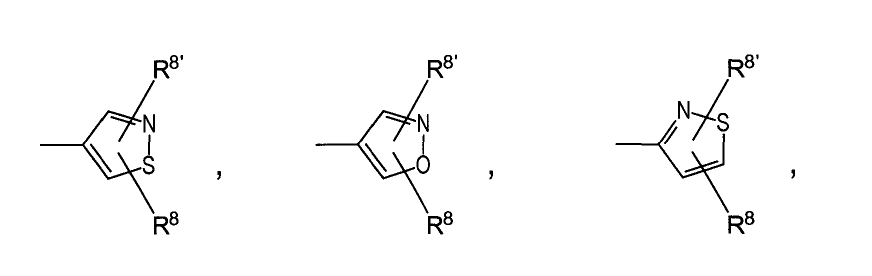 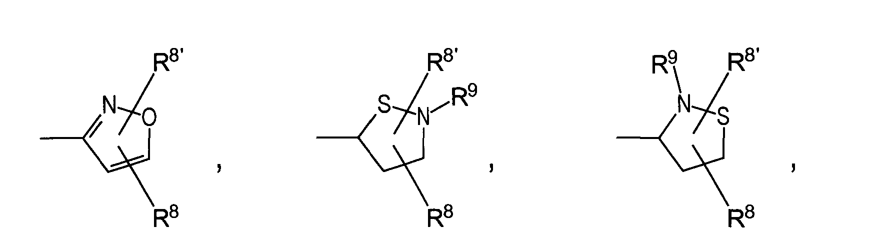 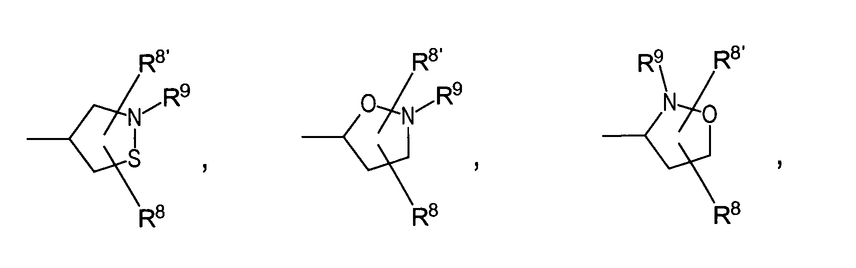 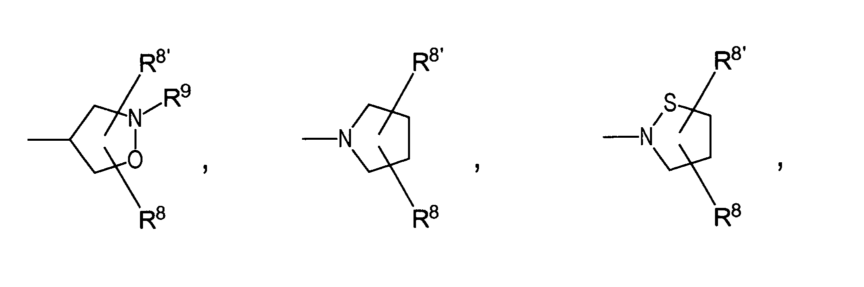 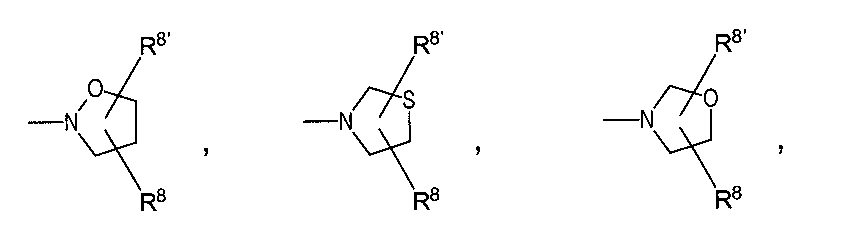 wherein R⁸ and R⁹ have the meanings as defined above; and R^{8'} represents independently of each other hydrogen, -COOR⁹, -CONHR⁹, -F, -Cl, -Br, -I, -NO₂, -CO-R⁹, -SO₂NHR⁹, -SO₂N(R⁹)₂, -NR⁹-CO-(C₁-C₆)-haloalkyl, -OH, -SH, -CN, -NR⁹-SO₂-(C₁-C₆)-haloalkyl, C₁-C₆-alkyl, C₁-C₆-aminoalkyl, C₁-C₆-alkoxy, C₁-C₆-alkylthio, C₁-C₆-hydroxyalkylamino, C₁-C₆-haloalkyloxy, C₅-C₁₅-aryl or heteroaryl.

The present invention provides also compounds of the following general formula (la) and pharmaceutically acceptable salts thereof, 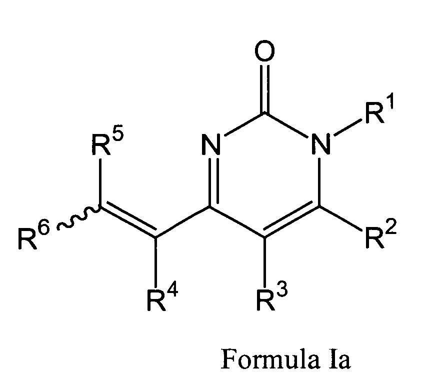 wherein:
R¹ represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷, -SO₂R⁷, -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰, R^{10'}, R^{10"} represents F, Cl, Br or I;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
with the proviso that when R⁵ or R⁶ are pyridine or phenyl and R² is methyl; and 4-(2,2-diphenylethenyl)-6-methyl-1*H*-pyrimdin-2-one are excluded, for the use as pharmaceutically active agent.

The C₁-C₆-alkyl, C₃-C₈-cycloalkyl, C₅-C₁₅-aryl, and heteroaryl group can optionally be substituted by one or more substituents R⁸.

R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;

R⁹ represents hydrogen, C₁-C₆-hydroxyalkyl, C₁-C₆-alkyl, C₁-C₆-aminoalkyl, C₅-C₁₅-aryl or heteroaryl.

A heteroaryl group represents a 5- or 6-membered heterocyclic group which contains at least one heteroatom like O, N, or S. This heterocyclic group can be fused to another ring. For example, this group can be selected from an oxazole-2-yl, oxazole-4-yl, oxazole-5-yl, thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo[b]furanyl, benzo[b]thiophenyl, benzimidazolyl, benzothiazolyl, quinazolinyl, quinoxazolinyl, preferably isoxazole-3-yl, isoxazole-4-yl, isoxazole-5-yl, quinolinyl, tetrahydroquinolinyl, isoquinolinyl, tetrahydroisoquinolinyl group. This heterocyclic group can optionally be substituted by one or more substituents R⁸, where R⁸ is as defined above.

The C₁-C₆-alkyl, C₁-C₆-alkenyl, C₁-C₆-alkinyl, and C₃-C₈-cycloalkyl residue may be selected from the group comprising -CH₃, -C₂H₅, -CH=CH₂, -C≡CH, -C₃H₇, -cyclo-C₃H₅, -CH(CH₃)₂, -CH₂-CH=CH₂, -C(CH₃)=CH₂, -CH=CH-CH₃, -C≡C-CH₃, -CH₂-C≡CH, -C₄H₉, -cyclo-C₄H₇, -CH₂-CH(CH₃)₂, -CH(CH₃)-C₂H₅, -C(CH₃)₃, -C₅H₁₁, -cyclo-C₅H₉, -C₆H₁₃, -cyclo-C₆H₁₁, -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇, -C₂H₄-C(R¹⁰)₃, -C₂H₄-CH=CH₂, -CH=CH-C₂H₅, -CH=C(CH₃)_{2,} -CH₂-CH=CH-CH₃, -CH=CH-CH=CH₂, -C₂H₄-C≡CH, -C≡C-C₂H₅, -CH₂-C≡C-CH_{3,} -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃ )-C₄H₉, -CH₂-CH(CH₃)-C₃H₇, -CH(CH₃)-CH₂-CH(CH₃)₂, -CH(CH₃ )-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C₃H₇, -C₂H₄-C≡C-C₂H₅, -cyclo-C₇H₁₃, -cyclo-C₈H₁₅ and R¹⁰, R^{10'} and R^{10"} represent independently of each other -H, -F, -Cl, -Br, -I.

The C₅-C₁₅-aryl represents -Ph, -CH₂Ph, -C₂H₄Ph, -CH=CH-Ph, -C≡C-Ph, -o-C₆H₄-R²,- -m-C₆H₄-R², -p-C₆H₄-R₂, -o-CH₂-C₆H₄-R², -m-CH₂-C₆H₄-R², -p-CH₂-C₆H₄-R², wherein R² is as defined above.

In another particularly preferred embodiment of the invention, in compounds of formula (I), R¹ is H, R² is CF₃ and R⁶ is furyl, which can optionally be substituted by one or more substituents R⁸, wherein R⁸ is as defined above.

In a further particularly preferred embodiment, in compounds of formula (I) , R¹ is H, R² is CF₂CI and R⁶ is furyl, which can optionally be substituted by one or more substituents R⁸, wherein R⁸ is as defined above.

In a further particularly preferred embodiment, in compounds of formula (I) , R¹ is H, R² is methyl and R⁶ is furyl, which can optionally be substituted by one or more substituents R⁸, wherein R⁸ is as defined above.

In another particularly preferred embodiment of the invention, in compounds of formula (I), R¹ is H, R⁴ is methyl, R² is CF₃ and R⁶ is phenyl, which can optionally be substituted by one or more substituents R⁸, wherein R⁸ is as defined above.

In a further particularly preferred embodiment, in compounds of formula (I) , R¹ is H, R² is CF₂CI, R⁴ is methyl and R⁶ is phenyl, which can optionally be substituted by one or more substituents R⁸, wherein R⁸ is as defined above.

In a further particularly preferred embodiment, in compounds of formula (I) , R¹ is H, R² is methyl, R⁴ is methyl and R⁶ is phenyl, which can optionally be substituted by one or more substituents R⁸, wherein R⁸ is as defined above.

In another particularly preferred embodiment of the invention, in compounds of formula (I), R¹ is H, R² is CF₃ and R⁶ is phenyl, which can optionally be substituted by one or more substituents R⁸, wherein R⁸ is as defined above.

In a further particularly preferred embodiment, in compounds of formula (I) , R¹ is H, R² is CF₂CI and R⁶ is phenyl, which can optionally be substituted by one or more substituents R⁸, wherein R⁸ is as defined above.

In a further particularly preferred embodiment, in compounds of formula (I) , R¹ is H, R² is methyl and R⁶ is phenyl, which can optionally be substituted by one or more substituents R⁸, wherein R⁸ is as defined above.

In another particularly preferred embodiment of the invention, in compounds of formula (I), R¹ is H, R² is CF₃ and R⁶ is naphthyl, which can optionally be substituted by one or more substituents R⁸, wherein R⁸ is as defined above.

In a further particularly preferred embodiment, in compounds of formula (I) , R¹ is H, R² is CF₂CI and R⁶ is naphthyl, which can optionally be substituted by one or more substituents R⁸, wherein R⁸ is as defined above.

In a further particularly preferred embodiment, in compounds of formula (I), R¹ is H, R² is methyl and R⁶ is naphthyl, which can optionally be substituted by one or more substituents R⁸, wherein R⁸ is as defined above.

Most preferred is the use of one or more compounds and/or pharmaceutically acceptable salts thereof, selected from the group comprising:
4-[2-(2-Bromo-5-methoxy-4-prop-2-ynyloxy-phenyl)-vinyl]-6-trifluoromethyl-1*H*-pyrimidine-2-one; 4-[2-(3-Chloro-4,5-diethoxyphenyl)-vinyl]-6-trifluoromethyl-1*H*-pyrimidine-2-one; 4-[2-(2,3-Dimethoxyphenyl)-vinyl]-6-trifluoromethyl-1*H*-pyrimidine-2-one; 4-[2-(2-Chloro-4-hydroxy-5-methoxy-phenyl)-vinyl]-6-trifluoromethyl-1*H*-pyrimidine-2-one; 4-{2-[4-(4-Fluorobenzyloxy)-phenyl]-vinyl}-6-trifluoromethyl-1*H*-pyrimidine-2-one; 4-[2-(2-Hydroxynaphtalen-1 -yl)-vinyl]-6-trifluoromethyl-1*H*-pyrimidine-2-one; 4-{2-[4-(2-Fluorobenzyloxy)-3-methoxyphenyl]-vinyl}-6-trifluoro-methyl-1*H*-pyrimidine-2-one; 4-[2-(4-Benzyloxy-3-ethoxyphenyl)-vinyl]-6-trifluoro-methyl-1*H*-pyrimidine-2-one; 4-[2-(3-Chloro-4,5-dimethoxy-phenyl)-vinyl]-6-trifluoro-methyl-1*H*-pyrimidine-2-one; 4-[2-(3-Hydroxyphenyl)-vinyl]-6-trifluoro-methyl-1*H*-pyrimidine-2-one; 4-{2-[4-(2-Chloro-6-fluorobenzyloxy)-3,5-dimethoxy-phenyl]-vinyl}-6-trifluoromethyl-1*H*-pyrimidine-2-one; 4-{2-[4-(2-Fluorobenzyloxy)-phenyl]-vinyl}-6-trifluoromethyl-1*H*-pyrimidine-2-one; 3-{5-[[2-(2-Oxo-6-trifouoromethyl-1,2-dihydro-pyrimidine-4-yl)-vinyl]-furan-2-yl}-benzoic acid methyl ester; 4-{2-[5-(2,3-Dichloro-phenyl)-furan-2-yl]-vinyl}-6-trifluoromethyl-1*H*-pyrimidine-2-one; 4-[2-(2-Chloro-5-nitrophenyl)-vinyl]-6-trifluoromethyl-1*H*-pyrimidine-2-one; 4-{2-[5-(2,5-Dimethyl-4-nitrophenyl)-furan-2-yl]-vinyl}-6-trifluoromethyl-1*H*-pyrimidine-2-one; 4-{2-[5-(2-Chloro-4-nitrophenyl)-furan-2-yl]-vinyl}-6-trifluoromethyl-1*H*-pyrimidine-2-one; 4-{2-[5-(1 -Oxo-1 ,3-dihydroisobenzofuran-5-yl)-furan-2-yl]-vinyl}-6-trifluoro-methyl-1*H*-pyrimidine-2-one; 4-[2-(4-Chlorophenyl)-vinyl]-6-trifluoromethyl-1*H*-pyrimidine-2-one; 4-[2-(2,4-Difluorophenyl)-vinyl]-6-trifluoromethyl-1*H*-pyrimidine-2-one; 4-[2-(3-Ethoxy-4-hydroxyphenyl)-vinyl]-6-trifluoromethyl-1*H*-pyrimidine-2-one; 4-[2-(4-Di-methylamino-3-nitrophenyl)-vinyl]-6-trifluoromethyl-1*H*-pyrimid ine-2-one; 4-[2-(4-Methoxyphenyl)-vinyl]-6-trifluoromethyl-1*H*-pyrimidine-2-one; 4-[2-(4-Hydroxy-5-methoxyphenyl)-vinyl]-6-trifluoromethyl-1*H*-pyrimidine-2-one; Carbonic acid ethyl ester 2 methoxy-4-[2-(2-oxo-6-trifluormethyl-1,2-dihydropyrimidine-4-yl)-vinyl]-phenyl ester; 4-[2-(4-Hydroxy-3,5-dimethoxyphenyl)-vinyl]-6-trifluoromethyl-1*H*-pyrimidine-2-one; 4-[2-(4-Hydroxyphenyl)-vinyl]-6-trifluoromethyl-1*H*-pyrimidine-2-one; 4-[2-(2-Hydroxy-4-methoxyphenyl)-vinyl]-6-trifluoromethyl-1*H*-pyrimidine-2-one; 4-[2-(3-Bromo-6-hydroxy-5-methoxyphenyl)-vinyl]-6-trifluoromethyl-1*H*-pyrimidine-2-one; 4-[2-(3,5-Dibromo-2-hydroxyphenyl)-vinyl]-6-trifluoromethyl-1*H*-pyrimidine-2-one; 4-[2-(2,6-Dichlorophenyl)-vinyl]-6-trifluoromethyl-1*H*-pyrimidine-2-one; 5-(4-Chlorophenyl)-2-[2-(2-oxo-6-trifluoromethyl-1,2-dihydro-pyrimidine-4-yl)-vinyl]-furan-3-carboxylic acid ethyl ester; 4-[2-(2-Trifluoromethylphenyl)-vinyl]-6-trifluoromethyl-1*H*-pyrimidine-2-one; 4-[2-(2-Oxo-6-trifluoromethyl-1,2-dihydropyrimidine-4-yl)-vinyl]-benzoic acid; 4-{2-[5-(2-Trifluoromehtylphenyl)-furan-2-yl]-vinyl}-6-trifluoromethyl-1*H*-pyrimidine-2-one; 5-{1 -[2-(2-Oxo-6-trifluoromethyl-1,2-dihydropyrimidine-4-yl)-vinyl]-naphtalene-2-yloxymethyl}-4,5-dihydro-isoxazole-3-carboxylic acid ethyl ester; 4-[2-(2-Allyloxynaphtalen-1-yl)-vinyl]-6-trifluoromethyl-1*H*-pyrimidine-2-one; 4-[2-(3-Phenylbenzo[c]isoxazol-5-yl )-vinyl]-6-trifluoromethyl-1*H*-pyrimidine-2-one; 4-{2-[2-(2-Oxo-6-trifluoromethyl-1,2-dihydropyrimidine-4-yl)-vinyl]-phenyl}-piperazine-1-carboxylic acid ethyl ester; 6-Trifluoromethyl-4-2-{2-[4-(5-trifluoromethyl-pyridine-2-yl )-piperazin-1-yl]-phenyl}-vinyl-1*H*-pyrimidine-2-one; 4-(2-[2-(4-Benzyl-piperazin-1-yl)-phenyl]-vinyl}-6-trifluoromethyl-1*H*-pyrimidine-2-one; 2-Methyl-5-{5-[2-(2-Oxo-6-trifouoromethyl-1,2-dihydro-pyrimidine-4-yl)-vinyl]-furan-2-yl}-benzoic acid; 4-{2-[5-(3-Chloro-4-methylphenyl)-furan-2-yl]-vinyl}-6-trifluoromethyl-1*H*-pyrimidine-2-one; 4-[2-(9-Ethyl-9*H*-carbazol-3-yl)-vinyl]-6-trifluoromethyl-1*H*-pyrimidine-2-one; 4-{2-[5-(4-Bromophenyl)-furan-2-yl]-vinyl}-6-trifluoromethyl-1*H*-pyrimidine-2-one; 4-(2-Benzo[1 ,3]dioxol-5-yl-vinyl)-6-trifluoromethyl-1*H*-pyrimidine-2-one; 4-[2-(3,4,5-Trimethoxyphenyl)-vinyl]-6-trifluoromethyl-1*H*-pyrimidine-2-one; 4-[2-(4-Chlorobenzo[1,3]dioxol-5-yl)-vinyl]-6-trifluoromethyl-1*H*-pyrimidine-2-one; 4-{2-[5-(3-Chlorophenyl)-furan-2-yl]-vinyl}-6-trifluoromethyl-1*H*-pyrimidine-2-one; 4-[2-(4-Pyrrolidin-1-yl-phenyl )-vinyl]-6-trifluoromethyl-1*H*-pyrimidine-2-one; 4-{2-[5-(4-Methoxy-2-nitrophenyl)-furan-2-yl]-vinyl}-6-trifluoromethyl-1*H*-pyrimid ine-2-one; 4-{2-[5-(3-Methyl-4-nitrophenyl)-furan-2-yl]-vinyl}-6-trifluoromethyl-1*H*-pyrimidine-2-one; 4-[2-(4-Trifluoromethylphenyl)-vinyl]-6-trifluoro-methyl-1*H*-pyrimidine-2-one; 4-(2-Furan-2-yl)-vinyl-6-trifluoromethyl-1H-pyrimidine-2-one; 4-{2-[5-(2-Chlorophenyl)-furan-2-yl]-vinyl}-6-trifluoromethyl-1*H*-pyrimidine-2-one; 4-{2-[5-(3-Nitrophenyl)-furan-2-yl]-vinyl}-6-trifluoromethyl-1*H*-pyrimidine-2-one; 4-{2-[5-(4-Chlorophenyl)-furan-2-yl]-vinyl}-6-trifluoromethyl-1*H*-pyrimidine-2-one; 4-{2-[5-(2-Nitrophenyl)-furan-2-yl]-vinyl}-6-trifluoromethyl-1*H*-pyrimidine-2-one; 4-[2-(5-Hydroxymethyl-furan-2-yl)-vinyl]-6-trifluoromethyl-1*H*-pyrimidine-2-one; 4-[2-(5-Methylthiophen-2-yl)-vinyl]-6-trifluoromethyl-1*H*-pyrimidine-2-one; 4-(2-Thiophen-2-yl)-vinyl-6-trifluoromethyl-1*H*-pyrimidine-2-one; 4-[2-(4-Bromo-5-tert-butylthiophen-2-yl)-vinyl]-6-trifluoromethyl-1*H*-pyrimidine-2-one; 4-{2-[3-(2,4-Dichlorobenzyloxy)-naphtalen-2-yl]-vinyl}-6-trifluoromethyl-1*H*-pyrimidine-2-one; 4-{2-[5-(2-Chloro-5-trifluoromethyl-phenyl)-furan-2-yl]-vinyl}-6-trifluoromethyl-1*H*-pyrimidine-2-one; 4-[2-(5-Bromofuran-2-yl)-vinyl]-6-trifluoromethyl-1*H*-pyrimidine-2-one; 4-(2-Benzofuran-2-yl-vinyl)- 6-trifluoromethyl-1*H*-pyrimidine-2-one; 4-[2-(5-Bromothiophen-2-yl)-vinyl]-6-trifluoromethyl-1*H*-pyrimidine-2-one; 4-[2-(5-Methylthiophen-2-yl)-vinyl]-6-trifluoromethyl-1*H*-pyrimidine-2-one; 2-[2-(2-oxo-6-trifluoromethyl-1 ,2-dihydro-pyrimidine-4-yl)-vinyl]-thiophene-3-carboxylic acid; 4-[2-(3-Hydroxynaphtalen-2-yl)-1-methylvinyl]- 6-trifluoromethyl-1*H*-pyrimidine-2-one; 4-[2-(3-Hydroxynaphtalen-2-yl)-vinyl]- 6-phenyl-1*H*-pyrimidine-2-one; 4-[2-(4-Trifluoromethylphenyl)-1-methyl-vinyl]-6-trifluoromethyl-1*H*-pyrimidine-2-one; 6-(Chlorodifluoromethyl)-4-[2-(4-trifluoromethyl-phenyl)-vinyl]-1*H*-pyrimidine-2-one; 4-[2-(4-Trifluoromethylphenyl)-vinyl]-6-phenyl-1*H*-pyrimidine-2-one; 6-(Chlorodifluoromethyl)-4-[2-(3,5-Dibromo-6-hydroxy-phenyl)-vinyl]-1*H*-pyrimidine-2-one; 4-[2-(3,5-Dibromo-6-hydroxyphenyl)-vinyl]-6-phenyl-1*H*-pyrimidine-2-one; 4-[2-(2,6-Dichlorophenyl)-1 -methyl-vinyl]-6-trifluoromethyl-1*H*-pyrimidine-2-one; 6-(Chlorodifluoromethyl)-4-[2-(2,6-Dichlorophenyl)-vinyl]-1*H*-pyrimidine-2-one; 4-[2-(2 ,6-Dichlorophenyl)-vinyl]-6-phenyl-1H-pyrimidine-2-one; 4-[2-(3,5-Dibromo-6-hydroxyphenyl)-1-methyl-vinyl]-6-trifluoromethyl-1*H*-pyrimidine-2-one; 4-[2-(2-Trifluoromethylphenyl)-1 -methylvinyl]-6-trifluoromethyl-1*H*-pyrimidine-2-one; 6-(Chlorodifluoro-methyl)-4-[2-(2-trifluoromethylphenyl)-vinyl]-1*H*-pyrimidine-2-one; 4-[2-(2-Trifluoromethylphenyl)-vinyl]-6-phenyl-1*H*-pyrimidine-2-one; 4-[2-(2-Hydroxynapthalen-1 -yl)-vinyl]-pyrimidine-2-one; 4,6-Bis-[2-(3,5-dibromo-2-hydroxyphenyl)-vinyl]-pyrimidine-2-one; 6-[2-(6-Nitro-benzo[1,3]dioxol-5-yl)-vinyl]-4-trifluoromethyl-1*H*-pyrimidine-2-one; 6-Trifluoro-methyl-4-[3-(4-trifluoromethylphenyl)-vinyl]-1*H*-pyrimidine-2-one; 4-[2-(3,5-Dibromo-2-hydroxy-phenyl)-vinyl]- pyrimidine-2-one; 4-[2-(3-Hydroxynaphthalen-2-yl)-vinyl]-pyrimidine-2-one; 4-[2-(3,5-Dibromo-2-hydroxyphenyl)-vinyl]-6-methyl-pyrimidine-2-one; 4,6-Bis-[2-(5-bromo-2-hydroxy-3-methoxyphenyl)-vinyl]-pyrimidine-2-one; 4-{2-[4-(2,4-Dichlorobenzyloxy)-3-methoxy-phenyl]-vinyl}-6-tri-fluoromethyl-1*H*-pyrimidine-2-one; 4,6-Bis-[2-(4-isopropylphenyl)-vinyl]-pyrimidine-2-one; 4-[2-(5-methylthiophen-2-yl]-vinyl]-6-trifluoromethyl-1*H*-pyrimidine-2-one; 6-Trifluoromethyl-4-[2-(2-trifluoromethylphenyl)-vinyl]-1*H*-pyrimidine-2-one; 4,6-Bis-[2-(2-trifluoro-methylphenyl)-vinyl]-pyrimidine-2-one; 4-[2-(2,6-Dichlorophenyl)-vinyl]-6-methyl-pyrimidine-2-one; 4-[2-(2,6-Dichlorophenyl)-vinyl]-pyrimidine-2-one; 4-[2-(2-Trifluoro-methylphenyl)-vinyl]-pyrimidine-2-one; 4-[2-(5-Bromo-2-hydroxy-3-methoxyphenyl)-vinyl]-6-methylpyrimidine-2-one.

In addition, the present invention provides methods for preparing the vinylpyrimidine-2-one of the general formula (I) and the vinylpyrimidine-2-one of the general formula (la). This step is described for example in D. M. Brown and G. A. R. Kon, *Journal of the Chemical Society* 1948, 2147-54.

One possibility for the synthesis of compounds of formula (I) comprises the step of reacting a pyrimidone of formula (II) with a carbonyl compound of formula (III).

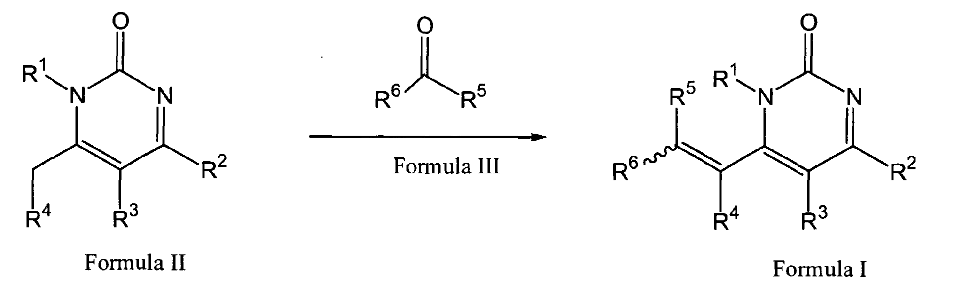

One possibility for the synthesis of compounds of formula (la) comprises the step of reacting a pyrimidone of formula (IIa) with a carbonyl compound of formula (III).

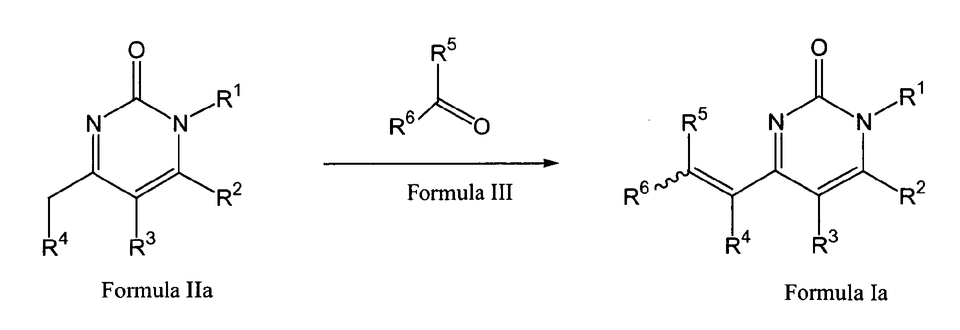

One method for preparing pyrimidones of formula (II) or formula (IIa) comprises the step of reacting a 1,3-diketone of formula (IV) with a urea of formula (V).

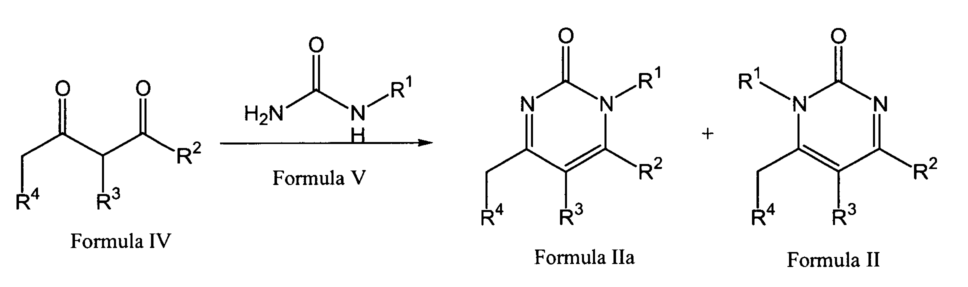

This step is described for example by S. Dilli and K. Robarts, *Australian Journal of Chemistry* 1978, 31, 1833-7; Ka *et al., European Journal of Medicinal Chemistry* 1995, 30, 423-7; H. G. Bonacorso *et al., Journal of Fluorine Chemistry* 1999, 99, 177-82; K. Mitsuhashi *et al., Heterocycles 1994,* 37, 1141-1146.

These pyrimidones of formula (II) or formula (lla) can be separated by chromatography, for example by column chromarography on silica gel [ A. Katoh et al., Chem. Pharm. Bull. 1994, 42(7), 1514-1517; C. Kashima, A. Katoh, J. Heterocyclic Chem. 1980, 17, 913-915].

Other aspects of the present invention relate to vinylpyrimidine-2-one derivatives of the general formula (I) or vinylpyrimidine-2-one derivatives of the general formula (la) as new pharmaceutically active agents, especially for the preparation of a pharmaceutical composition for the treatment of diseases which are cured or relieved by the inhibition of kinases and/or phosphatases. The compounds of the general formula (I) or compounds of the general formula (la) were surprisingly identified as potent inhibitors for human and viral kinases, especially of herpesviral kinases such as UL97.

Thus, a method is disclosed herein for preventing and/or treating diseases which are cured or relieved by the inhibition of kinases and/or phosphatases in a mammal, including a human, which method comprises administering to the mammal an amount of at least one compound of general formula (I) or compounds of the general formula (la) and/or pharmaceutically acceptable salts thereof, effective to prevent and/or treat said diseases which are cured or relieved by the inhibition of kinases and/or phosphatases. A preferred embodiment of said method involves the herpesviral kinase UL97 as a target for preventing and/or treating herpesviral infections and diseases.

Another method is directed to the prophylaxis and/or treatment of infectious diseases, including opportunistic infections in a mammal, including a human. Said method comprises administering to the mammal an amount of at least one compound of the general formula (I) or compounds of the general formula (la) and/or pharmaceutically acceptable salts thereof, effective to prevent and/or treat said infectious disease and/or opportunistic infection.

The infectious disease can be selected from the group comprising AIDS, Alveolar Hydatid Disease (AHD, Echinococcosis), Amebiasis (Entamoeba histolytica Infection), Angiostrongylus Infection, Anisakiasis, Anthrax, Babesiosis (Babesia Infection), Balantidium Infection (Balantidiasis), Baylisascaris Infection (Raccoon Roundworm), Bilharzia (Schistosomiasis), Blastocystis hominis Infection (Blastomycosis), Boreliosis, Botulism, Brainerd Diarrhea, Brucellosis, BSE (Bovine Spongiform Encephalopathy), Candidiasis, Capillariasis (Capillaria Infection), CFS (Chronic Fatigue Syndrome), Chagas Disease (American Trypanosomiasis), Chickenpox (Varicella-Zoster virus), Chlamydia pneumoniae Infection, Cholera, Chronic Fatigue Syndrome, CJD (Creutzfeldt-Jakob Disease), Clonorchiasis (Clonorchis Infection), CLM (Cutaneous Larva Migrans, Hookworm Infection), Coccidioidomycosis, Conjunctivitis, Coxsackievirus A16 (Hand, Foot and Mouth Disease), Cryptococcosis, Cryptosporidium Infection (Cryptosporidiosis), Culex mosquito (Vector of West Nile Virus), Cutaneous Larva Migrans (CLM), Cyclosporiasis (Cyclospora Infection), Cysticercosis (Neurocysticercosis), Cytomegalovirus Infection (CMV), Dengue / Dengue Fever, Dipylidium Infection (Dog and Cat Flea Tapeworm), Ebola Virus Hemorrhagic Fever, Echinococcosis (Alveolar Hydatid Disease), Encephalitis, Entomoeba coli Infection, Entomoeba dispar Infection, Entamoeba hartmanni Infection, Entamoeba histolytica Infection (Amebiasis), Entamoeba polecki Infection, Enterobiasis (Pinworm Infection), Enterovirus Infection (Non-Polio), Epstein-Barr Virus Infection, Escherichia coli Infection, Foodborne Infection, Foot and mouth Disease, Fungal Dermatitis, Gastroenteritis, Group A streptococcal Disease, Group B streptococcal Disease, Hansen's Disease (Leprosy), Hantavirus Pulmonary Syndrome, Head Lice Infestation (Pediculosis), Helicobacter pylori Infection, Hematologic Disease, Hendra Virus Infection, Hepatitis, Herpes Zoster (Shingles), HIV Infection, Human Ehrlichiosis, Human Parainfluenza Virus Infection, Influenza, Isosporiasis (Isospora Infection), Lassa Fever, Leishmaniasis, Kala-azar (Kala-azar, Leishmania Infection), Leprosy, Lice (Body lice, Head lice, Pubic lice), Lyme Disease, Malaria, Marburg Hemorrhagic Fever, Measles, Meningitis, Mosquito-borne Diseases, Mycobacterium avium Complex (MAC) Infection, Naegleria Infection, Nosocomial Infections, Nonpathogenic Intestinal Amebae Infection, Onchocerciasis (River Blindness), Opisthorciasis (Opisthorcis Infection), Parvovirus Infection, Plague, PCP (Pneumocystis carinii Pneumonia), Polio, Q Fever, Rabies, Respiratory Syncytial Virus (RSV) Infection, Rheumatic Fever, Rift Valley Fever, River Blindness (Onchocerciasis), Rotavirus Infection, Roundworms Infection, Salmonellosis, Salmonella Enteritidis, Scabies, Shigellosis, Shingles, Sleeping Sickness, Smallpox, Streptococcal Infection, Tapeworm Infection (Taenia Infection), Tetanus, Toxic Shock Syndrome, Tuberculosis, Ulcers (Peptic Ulcer Disease), Valley Fever, Vibrio parahaemolyticus Infection, Vibrio vulnificus Infection, Viral Hemorrhagic Fever, Warts, Waterborne infectious Diseases, West Nile Virus Infection (West Nile Encephalitis), Whooping Cough, Yellow Fever.

This relationship between a herpesviral kinase such as UL97 and the effective treatment of herpesviral infections led to the conclusion that the signal transduction mediated by a herpesviral kinase such as UL97 is essential for virus replication. Inhibition of said kinase, therefore, may decrease or inhibit the replication of herpes viruses. Surprisingly, it was found that the vinylpyrimidine-2-one derivatives of the general formula (I) or vinylpyrimidine-2-one derivatives of the general formula (la) act as potent inhibitors of a herpesviral kinase such as UL97 and the effect of decreasing up to preventing virus replication was observed.

Thus, a method for preventing and/or treating herpesviral infections and/or associated diseases in a mammal, including a human, is disclosed herein. Said method comprises the administration of a pharmaceutically effective amount of at least one compound of general formula (I) or compounds of the general formula (la) to said mammal, wherein said compound inhibits at least partially the activity of the herpesviral kinase UL97.

It can be stated that the vinylpyrimidine-2-one derivatives of the general formula (I) or vinylpyrimidine-2-one derivatives of the general formula (la) can be used for prophylaxis and/or treatment of infectious diseases, including opportunistic infections, especially of herpes and diseases caused by herpes viruses.

Herpesviruses may be selected from the group comprising α-herpesviruses (Simplexvirus, Varicellavirus), β-herpesviruses (Cytomegalovirus also known as human herpesvirus 5, Muromegalovirus, Roseolovirus), or γ-herpesviruses (Lymphocryptovirus, Rhadinovirus). Examples for α-herpesviruses are Herpes simplex virus type 1 (human herpesvirus 1), Herpes simplex virus type 2 (human herpesvirus 2), Varicella Zoster virus (human herpesvirus 3). Examples for γ-herpesviruses are Epstein-Barr virus (human herpesvirus 4) or human herpesvirus type 8 (HHV8). More preferably, the herpesvirus is Herpes simplex virus type 1, or Varicella Zoster virus, or Epstein-Barr virus (EBV), or human cytomegalovirus (HCMV), or human herpesvirus 6, or human herpesvirus 7, or human herpesvirus type 8 (HHV8). Most preferably, the herpesvirus represents the α-herpesviruses Herpes simplex virus type 1, or Varicella Zoster virus, or the γ-herpesviruses Epstein-Barr virus, or Human Herpes virus type 8.

The herpesvirus family comprises the human herpesviruses 1 to 8 and different herpes viruses for various animal species as shown below in Table 1:

**Table 1:**

| Members of the herpesvirus family | | | |
|---|---|---|---|
| **Subfamily** | **Genus** | **Human** | **Animal** |
| □-herpesvirus | simplex virus | human herpesvirus 1 (herpes simplex virus 1) | bovine herpesvirus 2 |
| | | human herpesvirus 2 (herpes simplex virus 2) | cercopithecine herpesvirus 1, (herpes B virus) |
| | varicella virus | human herpesvirus 3 (Varicella Zoster virus) | pseudorabiesvirus |
| | | | bovine herpesvirus 1 equine-abortion virus |
| □-herpesvirus | cytomegalovirus | human herpesvirus 5 (HCMV) | |
| | muromegalovirus | | murine herpesvirus 1 |
| | Roseolovirus | human herpesvirus 6, human herpesvirus 7 | aotine herpesvirus 1, 3 |
| □-herpesvirus | lymphocrytovirus | human herpesvirus 4 (Epstein-Barr virus) | cercopithecine herpesvirus 2 pongine herpesvirus 1 |
| | Rhadinovirus | human herpesvirus 8 | ateline herpesvirus 2 saimirine herpesvirus 1 |

Preferably, the herpes virus is selected from the group comprising herpes simplex viruses, varicello viruses, cytomegalo viruses, muromegalo viruses, roseolo viruses, lymphocrypto viruses, and rhadino viruses.

In the following some important members of the herpes virus family are described.

### Herpes simplex virus type 1 (HSV-1)

Herpes simplex virus is a highly adapted human pathogen with an extremely rapid lytic replication cycle, and yet with the ability to invade sensory neurons where highly restricted gene expression occurs with the absence of cytopathology.

Such latent infections are subject to reactivation whereby infectious virus can be recovered in peripheral tissue enervated by the latently infected neurons following a specific physiological stress. A major factor in these "switches" from lytic to latent infection and back involves the interaction between viral promoters, the viral genome, and cellular transcriptional machinery. Herpes simplex virus types 1 and 2 are generally associated with mucocutaneous facial infection (herpes labialis), genital infection (herpes genitalis), ophthalmic infection (herpes keratitis) and neonatal infection. The lesions are generally self-healing, but the infection remains, with the virus establishing latent infection in the neurons. In immunocompromised hosts, recurrence of HSV-1 may cause progressive fulminant lesions that are refractory to current antiviral therapy. Neonatal infection is usually systematically spread and may lead to encephalitis. Current antiviral therapy like Acyclovir does not eliminate viral infection. New therapies are needed to either eliminate latent virus or to suppress lesion development over the long term.

### Epstein-Barr virus (EBV)

Epstein-Barr virus, frequently referred to as EBV, is a member of the herpesvirus family and one of the most common human viruses. The virus occurs worldwide, and most people become infected with EBV sometime during their lives. In the United States, as many as 95% of adults between 35 and 40 years of age have been infected. Infants become susceptible to EBV as soon as maternal antibody protection (present at birth) disappears. In the United States and in other developed countries, many persons are not infected with EBV in their childhood years. When infection with EBV occurs during adolescence or young adulthood, it causes infectious mononucleosis 35% to 50% of the time.

Symptoms of infectious mononucleosis are fever, sore throat, and swollen lymph glands. Sometimes, a swollen spleen or liver involvement may develop. Heart problems or involvement of the central nervous system occurs only rarely, and infectious mononucleosis is almost never fatal. There are no known associations between active EBV infection and problems during pregnancy, such as miscarriages or birth defects. Although the symptoms of infectious mononucleosis usually resolve in 1 or 2 months, EBV remains dormant or latent in a few cells in the throat and blood for the rest of the person's life. Periodically, the virus can reactivate and is commonly found in the saliva of infected persons. This reactivation usually occurs without symptoms of illness.

EBV also establishes a lifelong dormant infection in some cells of the body's immune system. A late event in a very few carriers of this virus is the emergence of Burkitt's lymphoma and nasopharyngeal carcinoma, two rare cancers that are not normally found in the United States. EBV appears to play an important role in these malignancies, but is probably not the sole cause of disease. No antiviral drugs or vaccines for EBV except Ganciclovir are available.

### Human herpesvirus 8 (HHV8), or Kaposi's sarcoma-associated herpesvirus (KSHV)

KSHV is a virus belonging to the family of herpesviruses. KSHV was discovered in 1994. The virus has two names that are commonly used: KSHV which is its descriptive name and HHV8 which is its formal name. Both names are essentially interchangeable. KSHV causes a blood vessel cancer called Kaposi's sarcoma (KS), a lymphoma (a cancer of the lymphocyte) called body cavity-based lymphoma and some forms of severe lymph node enlargement, called Castleman's disease. The rates of KSHV infection in the general population of Mediterranean countries (Italy, Greece, Israel, Saudi Arabia) are higher than in North America and Northern Europe. Adult populations in some portions of Africa have very infection rates (>50%). This virus can be transmitted through sexual contact. KSHV is also probably transmitted to some extent through nonsexual routes, perhaps from oral contact (kissing). Once exposed to KSHV, infection is probably life-long and the immune system of healthy adults keeps the virus in check at extremely low levels. The real problem with KSHV occurs when an infected person becomes immunosuppressed. This occurs among transplant patients and patients receiving chemotherapy. There is good evidence that if someone is infected with KSHV and becomes immunosuppressed, then their chances of developing clinically detectable disease due to KSHV is higher than for other common viral infections. Infection with KSHV is diagnosed by a blood test. It now seems likely that early in the AIDS epidemic there were actually two simultaneous virus epidemics: the well-recognized epidemic of HIV and a second 'silent' epidemic of KSHV. Only those persons who became immunosuppressed by HIV developed KS. KSHV is probably less transmissible than HIV, certainly this is true from blood products and sharing needles, and so only the fraction of persons developing AIDS were infected with KSHV and developed KS. KSHV-related disease can also occur in persons without obvious immunodeficiency, but this is rare and primarily occurs among elderly men. There has been a major epidemic of KS in some parts of Africa, especially Eastern and Southern Africa, concurrent with the African HIV pandemic. KS is now the most common tumor reported in several African cancer registries. While most African KS patients are also HIV infected, these areas also have the world's highest rates of this disease for non HIV-infected persons. There is no vaccine for KSHV. Existing therapy is Ganciclovir.

### Varicella-zoster virus (VZV)

Chicken pox is an infection that is caused by the Varicella-zoster virus (VZV). VZV spreads in nasal discharge and in fluid from inside the chicken pox blisters. Chicken pox is very contagious, and 90% of people who are not immune will catch it when they are exposed. Epidemics are most common in the late winter and early spring, and children between ages 5 and 9 account for half of all cases.

Normally, chicken pox is a mild illness, but it can cause serious complications, including pneumonia, encephalitis, and serious bacterial infections of chicken pox blisters. After causing an attack of chicken pox, VZV remains in the body. It lies dormant in nerve cells and may be reactivated later in life to cause shingles. Children (and others) who develop varicella and have weakened immune systems may be treated with Acyclovir.

Having identified the herpesviral kinase UL97 as potent target for prophylaxis and/or treatment of herpesviral infections and/or diseases associated with herpesviral infections a method for the identification of potent inhibitior for UL97 has been designed. Said method comprises contacting a test compound with the herpesviral kinase UL97 and detecting a change in activity of said cellular kinase. Potent inhibitors such as the vinylpyrimidin-2-one derivatives of the general formula (I) will markedly diminish the activity of the herpesviral kinase UL97.

Still another aspect of the present invention is related to a method for detecting herpesviral infections and/or associated diseases in an individual comprising:
a) providing a sample from said individual; and
b) determining activity in said sample of the herpesviral kinase UL97.

A similar method for detecting herpesviral infections and/or associated diseases in cells, cell cultures and/or cell lysates is disclosed. Said method comprises:
a) providing a sample from said cells and/or cell lysates; and
b) determining activity in said sample of the herpesviral kinase UL97.

Beside herpesviral infections and diseases associated with herperviral infections the vinylpyrimidine-2-one compounds can also be used for the manufacture of a pharmaceutical formulation for prophylaxis and/or treatment of diseases like cell proliferation disorders, cardiovascular disorders, immunological diseases, inflammatory diseases, neuroimmunological diseases, autoimmune diseases.

Furthermore, the present invention refers to a method for preventing and/or treating diseases like cell proliferation disorders, cardiovascular disorders, immunological diseases, inflammatory diseases, neuroimmunological diseases, autoimmune diseases preferably in mammal, most preferably in human. Said method comprises administering to the mammal an amount of at least one vinylpyrimidine-2-one derivative of the general formula (I) or one vinylpyrimidine-2-one derivative of the general formula (la) and/or pharmaceutically acceptable salts thereof, effective to prevent and/or treat cell proliferation disorders, cardiovascular disorders, immunological diseases, inflammatory diseases, neuroimmunological diseases, and/or autoimmune diseases.

The compounds of the present invention are also useful for the treatment of diseases which are caused by malignant cell proliferation, such as all forms of hematological and solid cancer. Therefore the compounds according to the invention and pharmaceutical compositions prepared therewith are generally useful for regulating cell activation, cell proliferation, cell survival, cell differentiation, cell cycle, cell maturation and cell death or to induce systemic changes in metabolism such as changes in sugar, lipid or protein metabolism.

They can also be used to support cell generation poiesis, including blood cell growth and generation (prohematopoietic effect) after depletion or destruction of cells, as caused by, for example, toxic agents, radiation, immunotherapy, growth defects, malnutrition, malabsorption, immune dysregulation, anemia and the like or to provide a therapeutic control of tissue generation and degradation, and therapeutic modification of cell and tissue maintenance and blood cell homeostasis.

The compounds according to the invention and pharmaceutical compositions prepared therewith are generally useful for the treatment of cell proliferation disorders, for the treatment or prophylaxis of cardiovascular disorders, immunological diseases and conditions (as for instance inflammatory diseases, neuroimmunological diseases, autoimmune diseases or other).

These diseases and conditions include but are not limited to cancer as hematological (e.g. leukemia, lymphoma, myeloma) or solid tumors (for example breast, prostate, liver, bladder, lung, esophageal, stomach, colorectal, genitourinary, gastrointestinal, skin, pancreatic, brain, uterine, colon, head and neck, ovarian, melanoma, astrocytoma, small cell lung cancer, glioma, basal and squameous cell carcinoma, sarcomas as Kaposi's sarcoma and osteosarcoma), tumor angiogenesis or metastasis, treatment of disorders involving T-cells such as acute or chronic graft rejection or other host versus graft or graft versus host reactions, aplastic anaemia and DiGeorge syndrome, Graves' disease, lupus erythematosus, Sjogren's Syndrome, fibrosis, uveitis, rhinitis, asthma or athropathy, in particular, arthrosis, all forms of rheumatism or arthritis as rheumatoid arthritis, rheumatoid spondylitis, osteoarthritis, gouty arthritis, multiple sclerosis, pancreatitis, glomerulonephritis, ulcerative colitis, sickle cell anaemia or other forms of anaemia progressive retinal atrophy, inflammatory bowel disease, Morbus Crohn, chronic pulmonary inflammatory disease as well as other chronic or acute inflammations or inflammatory diseases, chronic diarrhea, insulin dependent diabetes mellitus and non-insulin dependent diabetes mellitus as well as diabetic conditions as glaucoma, retinopathy or microangiopathy and other metabolic diseases, ocular conditions as ocular or macula edema, ocular neovascular disease, scleritis, radial keratomy, uveitis, vitritis, myopia, chronical retinal detachment, post-laser treatment complications, conjunctivitis, Stargardt's disease, Eales disease or retinopathy, dermatological disorders such as psoriasis and acute immunological events and disorders such as sepsis, septic shock, endotoxic shock, Gram-negative sepsis, toxic shock sydrome, acute respiratory distress syndrome, stroke, reperfusion injury, CNS injury, serious forms of allergy, Alzheimer's disease or pyresis, atherosclerosis, ischemia/reperfusion injury as for instance stroke or infarct, cardiovascular diseases, vascular smooth muscle proliferation conditions as postsurgical vascular stenosis, restenosis, angina pectoris chronic pulmonary inflammatory disease, silicosis, pulmonary sarcosis, fibrosis, Heppel-Lindau disease, gangrene, necrosis, benign prostate hyperplasia, bone resorption disease as osteoporosis.

The inventive vinylpyrimidine-2-one compounds of the general formula (I) or vinylpyrimidine-2-one compounds of the general formula (la) and/or pharmaceutically acceptable salts thereof are administered in a dosage corresponding to an effective concentration in the range of 0.01 - 50 µM, preferably in the range of 0.02 - 10 µM, more preferably in the range of 0.03 - 1 µM, and most preferably in the range of 0.04 - 0.1 µM.

In a further aspect the present invention relates to pharmaceutical compositions comprising at least one compound of the general formula (I) or one compound of the general formula (la) as an active ingredient together with one or more pharmaceutically acceptable carrier(s), excipient(s) and/or diluent(s).

The vinylpyrimidine-2-one compounds of the present invention can form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid and other mineral or carboxylic acids well known to those skilled in the art. The salts are prepared by contacting the free base form with a sufficient amount of the desired acid to produce a salt in the conventional manner.

It is also possible to obtain acid addition salts with amino acids like methionine, tryptophane, lysine or arginine, especially with vinylpyrimidine-2-one compounds of the general formula (I) or vinylpyrimidine-2-one compounds of the general formula (la) containing a carboxylic acid residue.

Depending upon the substituents on the inventive vinylpyrimidine-2-one compounds, one can form salts with bases too. Thus, for example, if there are carboxylic acid substituents in the molecule, salts may be formed with inorganic as well as organic bases such as, for example, NaOH, KOH, NH₄0H, tetraalkylammonium hydroxide, and the like.

The compounds of formula (I) or of formula (la) can also be used in the form of a precursor (prodrug) or a suitably modified form, that releases the active compound *in vivo.* Such precursors can be obtained for example by masking the free acid group with an ester group, which is then in turn transformed into the free acid group *in vivo* [F. W. Sum et. al. Bioorg. & Med. Chem. Lett. 9 (1999), 1921-1926; Ada, Rephaeli et. al. Drug Development Research 50 (2000) 379-391; H. Ishikawa, Current Med. Chem. 6 (1999), 575-597].

The compounds of the general formula (I) or compounds of the general formula (la) can also be administered in form of their pharmaceutically active salts optionally using substantially nontoxic pharmaceutically acceptable carriers, excipients or diluents. The medications of the present invention are prepared in a conventional solid or liquid carrier or diluents and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits.

Furthermore, the subject of the present invention also includes pharmaceutical preparations for parenteral, including dermal, intradermal, intragastrical, intracutaneous, intravasal, intravenous, intramuscular, intraperitoneal, intranasal, intravaginal, intrabuccal, percutaneous, rectal, subcutaneous, sublingual, topical or transdermal application, which in addition to typical vehicles and diluents contain a vinylpyrimidine-2-one compound of the general formula (I) or a vinylpyrimidine-2-one compound of the general formula (la) and/or a pharmaceutically acceptable salt thereof as active ingredient.

Within the disclosed methods the pharmaceutical compositions of the present invention, containing vinylpyrimidine-2-one derivatives of the general formula (I) or vinylpyrimidine-2-one compound of the general formula (la) as active ingredients, will typically be administered in admixture with suitable carrier materials selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethylcellulose, polyethylene glycol and waxes. Among the lubricants, there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive vinylpyrimidine-2-one compounds of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophilic semi-solid matrix.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight.

The term disintegrants refers to materials added to the composition to help it break apart (disintegrate). Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.

Binders characterize substances that bind or "glue" powders together and make them cohesive by forming granules, thus serving as the "adhesive" in the formulation. Binders add cohesive strength already available in the diluent or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D,L-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.

Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.1 to about 5% by weight of the composition, preferably from about 0.1 to about 1%.

### Examples:

### General synthesis of compounds of formula (I) or of formula (la)

A carbonyl compound (1-3 eq.) of formula (lll) and piperidine or concentrated hydrochloric acid (catalytic amounts) were added to a solution of a pyrimidone of formula (II) or formula (lla) (1 eq.) in ethanol. The mixture was refluxed for 16 to 72 h and concentrated in vacuum. The residue was purified by appropriate means, e.g. recrystallization, preparative thin layer chromatography (Merck, 20 x 20 cm, Silica gel 60 F₂₅₄, 1 mm) or preparative HPLC.

### General synthesis of compounds of formula (II) or of formula (IIa)

Concentrated hydrochloric or sulfuric acid was added to a mixture of a 1,3-dicarbonyl compound of formula (IV) (1 eq.) and a urea compound of formula (V) (1-3 eq.) in ethanol or ethanol / water (1 / 1) at ambient temperature. The mixture was refluxed for 5 to 18 h and concentrated in vacuum. The residue was purified by appropriate means, e.g. recrystallization, flash chromatography or prepeative HPLC.

### Synthesis of 4-Methyl-6-trifluoromethylpyrimidine-2-one

Concentrated sulfuric acid (0,5 ml) was added to a mixture of 1,1,1-trifluoropentane-2,4-dione (1000 mg, 6,5 mmol) and urea (780 mg, 13 mmol) in ethanol / water (1 / 1, 30 ml). The mixture was refluxed for 5 h and concentrated in vacuum to about half the volume. Ethyl acetate was added, the two layers separated and the aqueous layer was extracted with ethyl acetate three times. The combined organic layers were washed with saturated sodium bicarbonate solution, dried over sodium sulfate and concentrated in vacuum. The residue was purified by flash chromatography to yield 179 mg (15 %) of the title compound. Mass found: 179 [M + H], 177[M-H].

### Synthesis of 5-(4-Chlorophenyl)-2-[2-(2-oxo-6-trifluoromethyl-1,2-di hydropyrimidine-4-yl)-vinyl]-furan-3-carboxylic acid ethyl ester

Piperidine (2 drops) was added to a mixture of 5-(4-chlorophenyl)-2-formylfuran-3-carboxylic acid ethyl ester (31 mg, 0,11 mmol) and 4-methyl-6-trifluoromethylpyrimidine-2-one (20 mg, 0,11 mmol) in ethanol (3 ml). The mixture was refluxed for 24 h and then cooled to room temperature. The precipitate was filtered off, washed with ethanol and dried in vacuum to yield the title compound. Mass found: 439 [M + H], 437 [M - H].

### Synthesis of 4-[2-(2-Allyloxynaphthalen-1-yl)-vinyl]-6-trifluoromethyl-1H-pyrimidine-2-one

Piperidine (2 drops) was added to a mixture of 2-allyloxynaphthalene-1-carbaldehyde (10 mg, 0,05 mmol) and 4-methyl-6-trifluoromethylpyrimidine-2-one (8 mg, 0,05 mmol) in ethanol (3 ml). The mixture was refluxed for 16 h, cooled to room temperature and concentrated in vacuum. The residue was purified by preparative thin layer chromatography (eluent: ethyl acetate) to yield the title compound. Mass found: 373 [M + H], 371 [M - H].

### Synthesis of 4-{2-[5-(3-Methyl-4-nltrophenyl)-furan-2-yl]-vinyl}-6-trifluoromethyl-1H-pyrimidine-2-one

Piperidine (2 drops) was added to a mixture of 5-(3-methyl-4-nitrophenyl)-furan-2-carbaldehyde (10 mg, 0,05 mmol) and 4-methyl-6-trifluoromethylpyrimidine-2-one (8 mg, 0,05 mmol) in ethanol (3 ml). The mixture was refluxed for 16 h, cooled to room temperature and concentrated in vacuum. The residue was purified by preparative HPLC to yield the title compound. Mass found: 392 [M+H], 390 [M-H].

### Materials and methods

### UL97 Kinase-Assay on immobilon Plate

The effect of the vinylpyrimidine-2-one derivatives were tested on the activity of the viral kinase UL-97. This kinase is derived from human cytomegalovirus (HCMV) (Marschall, M., Stein-Gerlach, M., Freitag, M., Kupfer, R., van den Bogaard, M. & Stamminger, T.; Inhibitors of human cytomegalovirus replication drastically reduce the activity of the viral protein kinase pUL97. *J. Gen. Virol.* 2001, 82, 1439-1450.). The UL-97 gene was cloned into a baculovirus vector in order to produce GST (glutathione S-transferase) fusion protein. Insect cells (Sf9) were infected and GST-UL-97 purified via glutathione affinity columns according to standard procedures.

### UL-97 Kinase Reaction

The UL-97 kinase reaction was performed as described (Marschall *et al.,* 2001, 1439-1450). Briefly, 10 µl Assay buffer (3 µM ATP, 60µg/ml myelin basic protein (MBP) as substrate), 1,0 µCi □-[³³P]ATP and 10 µl Basic buffer (20mM Tris-HCI 7.5, 500µM MnCl_{2,} 1mM DTT (dithiothreitol)) were given to the test tube before adding various concentrations of the vinylpyrimidine-2-one derivatives. The reaction was started by adding 0.2 µl UL97 kinase, purified from infected Sf9-insect cells as described above. The total volume was adjusted with Basic buffer to a final volume of 30 µl. The reaction mix was incubated for 1Hr at 30°C. For negative control, 10 µl 0.1M EDTA (ethylene diamine tetraacetate) was added to reaction mix before addition of UL-97 protein kinase. The reaction was stopped by addition of 10 µl 0.1 M EDTA.

### Measuring Incorporation of Radioactivity

The Immobilon plate (Millipore) was rinsed with 50µl methanol/well. Following addition of 100 µl 0.1 M EDTA, 20 µl of each kinase reaction mix was added to one well of the Immobilon plate. Each well was washed 4x with 250µl 0.75% phosphoric acid and 1x with 50µl methanol. After addition of 50µl scintillation cocktail (Roth, Germany) per well incorporation of radioactivity was measured using a Betareader (Wallac) and enzymatic activity calculated.

### Results

i) The data show that representatives of the class of vinylpyrimidine-2-one derivatives (the following compounds: 4-[2-(2-Hydroxy-naphtalene-1-yl)-vinyl]-6-trifluoromethyl-1*H*-pyrimidine-2-one; 4-{2-[5-(2,3-Dichlorophenyl)-furan-2-yl]-vinyl}-6-trifluoromethyl-1*H*-pyrimidine-2-one; 4-{2-[5-(1-Oxo-1,3-dihydro-isobenzofuran-5-yl)-furan-2-yl]-vinyl}-6-trifluoro-methyl-1*H*-pyrimidine-2-one;4-[2-(2-Chloro-5-nitro-phenyl)-vinyl]-6-trifluoromethyl-1*H*-pyrimidine-2-one; 4-[2-(3,5-Dibromo-2-hydroxy-phenyl)-vinyl]-6-trifluoromethyl-1*H*-pyrimidine-2-one; 5-(4-Chlorophenyl)-2-[2-(2-oxo-6-trifluoromethyl-1,2-dihydro-pyrimidine-4-yl)-vinyl]-furan-3-carboxylic acid ethyl ester; 4-[2-(2-Trifluoromethylphenyl)-vinyl]-6-trifluoromethyl-1*H*-pyrimidine-2-one; 5-{1-[2-(2-Oxo-6-trifluoromethyl-1,2-dihydropyrimidine-4-yl)-vinyl]-naphtalene-2-yloxy-methyl}-4,5-dihydro-isoxazole-3-carboxylic acid ethyl ester; 4-{2-[5-(3-Chloro-4-methylphenyl)-furan-2-yl]-vinyl}-6-trifluoromethyl-1*H*-pyrimidine-2-one; 4-[2-(4-Tri-fluoromethylphenyl)-vinyl]-6-trifluoromethyl-1*H*-pyrimidine-2-one; are efficient inhibitors of pUL97 in a range of 3 - 30 µM.
ii) Compounds belonging to the class of vinylpyrimidine-2-one derivatives have been identified as potent inhibitors of HCMV replication *in vitro* with IC50 values as low as 3 µM and thus are as good as the gold standard ganciclovir (IC50 3-4 µM). Furthermore, compounds of the class of vinylpyrimidine-2-ones block pUL97 kinase activity at sub - and low micromolar concentrations.
   Their IC50 values for blocking the kinases p38, EGF-Rec., JNK, Abl, PKC, Akt, Ins.-Rec., c-kit, Lck, MAPK, c-met, p70S6, PDGF-Rec. and src. are far higher than 10 µM.
iii) The vinylpyrimidine-2-one derivatives are non-toxic up to concentrations of 10 µM in HEK293 (human embryonic kidney) cells and HFF (human foreskin fibroblasts) cells.

## Claims

1. Compounds of the general formula (I) and pharmaceutically acceptable salts thereof, 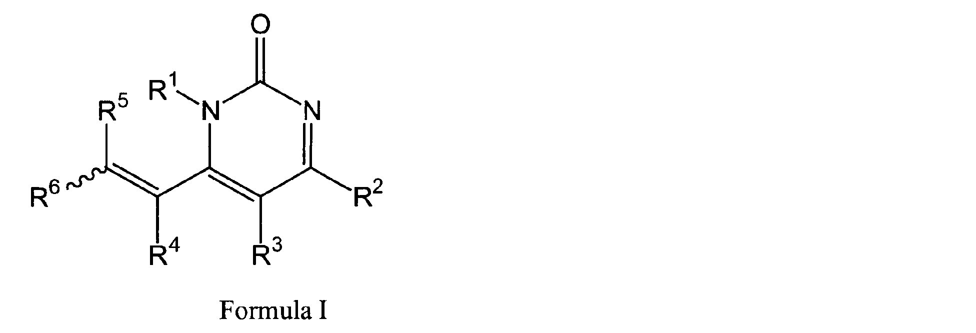 wherein:
R¹ represents C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷, -SO₂R⁷; -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰, R^{10'}, R^{10"} represents F, Cl, Br or I;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
with the proviso that 5-Chloro-1,2,3,6-tetrahydro-1-methyl-2-oxo-6-(2-oxopropyl)-4-pyrimidinecarboxylic acid methylester is excluded;
the C₁-C₆-alkyl, C₃-C₈-cycloalkyl, C₅-C₁₅-aryl, and heteroaryl group can optionally be substituted by one or more substituents R⁸;
R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;
R⁹ represents hydrogen, C₁-C₆-hydroxyalkyl, C₁-C₆-alkyl, C₁-C₆-aminoalkyl, C₅-C₁₅-aryl or heteroaryl.

2. Compounds of the general formula (la) and pharmaceutically acceptable salts thereof, 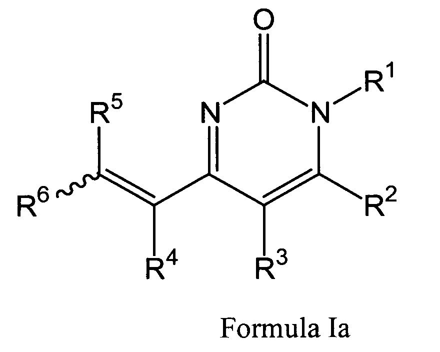 wherein:
R¹ represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₅-C₁₅-aryl or heteroaryl;
with the proviso that the following compounds are excluded:
4-(2,2-diphenylethenyl)-6-methyl-1*H*-pyrimidine-2-one; 4-methyl-6-(2-oxo-2-phenylethyl)-5-phenyl-1*H*-pyrimidine-2-one; 1,2-dihydro-4-methyl-2-oxo-6-(2-phenyl-ethenyl)-ethylester-5-pyrimidinecarboxylic acid; 2,3-dihydro-4-[2-(4-methoxy-phenyl)ethenyl]-1,3,6-trimethyl-2-oxo-pyridinium; 2,3-dihydro-4-[2-(4-methoxy-phenyl)ethenyl]-1,3,6-trimethyl-2-oxo-pyridinium perchlorate; 4-phenyl-6-(2-oxo-2-phenylethyl)-1*H*-pyrimidine-2-one; 4-(2,2-diphenylethenyl)-6-phenyl-1*H*-pyrimidine-2-one; 6-phenyl-4-styryl-1*H*-pyrimidine-2-one; 4-(1*H*-indol-2-yl)-6-[(1E)-2-(2-nitro-phenyl)ethenyl]-1*H*-pyrimidine-2-one; 4-[2-[4-[(2-cyanoethyl)methylamino]-phenyl]-ethenyl]-2,3-dihydro-1,3-dimethyl-2-oxo-pyrimidinium; (*E*)-5-chloro-4-(2-phenyl-ethenyl)-1-(phenylmethyl)-1*H*-pyrimidine-2-one; (*E*)-5-chloro-4-(2-phenylethenyl)-1*H*-pyrimidine-2-one; 4-[2-(4-bromophenyl)-2-oxoethylidene]-1-cyclohexyl-3,4-dihydro-1*H*-pyrimidine-2-one; (Z)-3,4-dihydro-4-(2-oxo-2-phenylethylidene)-1-(phenylmethyl)-1*H*-pyrimidine-2-one; (Z)-5-chloro-3,4-dihydro-1 -methyl-4-(2-oxo-2-phenyl-ethylidene)-1*H*-pyrimidine-2-one; (Z)-5-chloro-3,4-dihydro-4-(2-oxo-2-phenyl-ethylidene)-1-phenylmethyl-1*H*-pyrimidine-2-one; 4-[2-[4-(dimethylamino)-phenyl]-ethenyl]-2,3-dihydro-2-oxo-1,3-diphenylpyrimidinium perchlorate; 4-[2-[4-(di-methylamino)phenyl]ethenyl]-2,3-dihydro-2-oxo-1,3-diphenyl-pyrimidinium; 4-[2-(4-bromophenyl)-2-oxoethylidene]-3,4-dihydro-3-methyl-1*H*-pyrimidine-2-one; 4-[2-(4-chlorophenyl)ethenyl]-1*H*-pyrimidine-2-one; 4-[2-(4-bromophenyl)-2-oxoethylidene]-3,4-dihydro-1-methyl-1*H*-pyrimidine-2-one; 3,4-dihydro-4-[2-(4-methoxyphenyl)-2-oxoethylidene]-1*H*-pyrimidine-2-one; 3,4-dihydro-4-(2-oxo-2-phenylethylidene)-1*H*-pyrimidine-2-one; 4-[2-(4-bromophenyl)-2-oxoethylidene]-3,4-dihydro-1*H*-pyrimidine-2-one; 3,4-di-hydro-4-(2-oxo-2-phenylethylidene)-1-β-D-ribofuranosyl-1*H*-pyrimidine-2-one; 4-(2-hydroxy-2-phenylethenyl)-1 -β-D-ribofuranosyl-1*H*-pyrimidine-2-one; 4-(2-hydroxy-2-phenylethenyl)-1-(2,3,5-tri-O-benzoyl-β-D-ribo-furanosyl)-1*H*-pyrimidine-2-one; 2,3-dihydro-1,3-dimethyl-4-[2-[4-methyl-2-(methylthio)-5-thiazolyl]ethenyl]-2-oxo-pyrimidinium; 2,3-dihydro-1,3-di-methyl-4-[2-[4-methyl-2-(methylthio)-5-thiazolyl]ethenyl]-2-oxo-pyrimidinium perchlorate; 2,3-dihydro-1,3-dimethyl-4-[2-[2-(methylphenylamino)-4-phenyl-5-thiazolyl]ethenyl]-2-oxo-pyrimidinium; 4-[2-[2-(4-chlorophenyl)-1 -methyl-1*H*-indol-3-yl]ethenyl]-2,3-dihydro-1,3-dimethyl-2-oxo-pyrimidinium; 2,3-dihydro-1,3-dimethyl-4-[2-(1 -methyl-2-phenyl-1*H*-indol-3-yl)ethenyl]-2-oxopyrimidinium; 2,3-dihydro-1,3-dimethyl-4-[2-[4-methyl-2-(methylphenylamino)-5-thiazolyl]ethenyl]-2-oxo-pyrimid inium; 4-[2-[2-(dimethylamino)-4-phenyl-5-thiazolyl]ethenyl]-2,3-dihydro-1,3-dimethyl-2-oxo-pyrimidinium; 2,3-dihydro-1,3-dimethyl-4-[2-[2-(methylthio)-4-phenyl-5-thiazolyl]-ethenyl]-2-oxo-pyrimidinium; 4-[2-[2-(dimethylamino)-5-thiazolyl]ethenyl]-2,3-dihydro-1,3-dimethyl-2-oxo-pyrimidinium; 4-[2-[4-(dimethylamino)phenyl]-ethenyl]-2,3-dihydro-1,3-dimethyl-2-oxo-pyrimidinium; 2,3-dihydro-4-[2-(4-methoxy-phenyl)ethenyl]-1,3-dimethyl-2-oxo-pyrimidinium; 4-[2-[2-(dimethylamino)-5-thiazolyl]ethenyl]-2,3-dihydro-1,3-dimethyl-2-oxo-pyrimidinium perchlorate; 4-[(3-ethyl-4-oxo-2-thioxo-5-thiazolidinylidene)ethylidene]-3,4-dihydro-1,3-dimethyl-1*H*-pyrimidine-2-one; 4-[2-[2-(4-chlorophenyl)-1-methyl-1*H*-indol-3-yl]ethenyl]-2,3-dihydro-1,3-dimethyl-2-oxo-pyrimidinium perchlorate; 2,3-dihydro-1,3-dimethyl-4-[2-(1 -methyl-2-phenyl-1*H*-indol-3-yl)ethenyl]-2-oxo-pyrimidinium perchlorate; 2,3-dihydro-1,3-dimethyl-4-[2-[4-methyl-2-(methylphenylamino)-5-thiazolyl]-ethenyl]-2-oxo-pyrimidinium perchlorate; 2,3-dihydro-1 ,3-dimethyl-4-[2-[2-(methyl-phenylamino)-4-phenyl-5-thiazolyl]ethenyl]-2-oxo-pyrimidinium perchlorate; 4-[2-[2-(dimethylamino)-4-phenyl-5-thiazolyl]ethenyl]-2,3-dihydro-1,3-dimethyl-2-oxo-pyrimidinium perchlorate; 2,3-dihydro-1,3-dimethyl-4-[2-[2-(methylthio)-4-phenyl-5-thiazolyl]ethenyl]-2-oxo-pyrimidinium perchlorate; 4-[2-[4-(dimethylamino)-phenyl]-ethenyl]-2,3-dihydro-1,3-dimethyl-2-oxo-pyrimidinium perchlorate; 2,3-dihydro-4-[2-(4-methoxyphenyl)ethenyl]-1,3-dimethyl-2-oxo-pyrimidinium perchlorate; 4-(p-chlorophenacylidene)-3,4-dihydro-1,3-dimethyl-1*H*-pyrimidine-2-one; 3,4-dihydro-1,3-dimethyl-4-(p-nitrophenacylidene)-1*H*-pyrimidine-2-one; 1,2-dihydro-2-oxo-4-(2-hydrazono-2-phenylethyl)-5-pyrimidinecarboxylic acid hydrazide; 1,2-dihydro-2-oxo-6-styryl-5-pyrimidinecarboxylic acid;
the C₁-C₆-alkyl, C₃-C₈-cycloalkyl, C₅-C₁₅-aryl, and heteroaryl group can optionally be substituted by one or more substituents R⁸;
R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;
R⁹ represents hydrogen, C₁-C₆-hydroxyalkyl, C₁-C₆-alkyl, C₁-C₆-aminoalkyl, C₅-C₁₅-aryl or heteroaryl.

3. Compounds of the general formula (la) and pharmaceutically acceptable salts thereof, 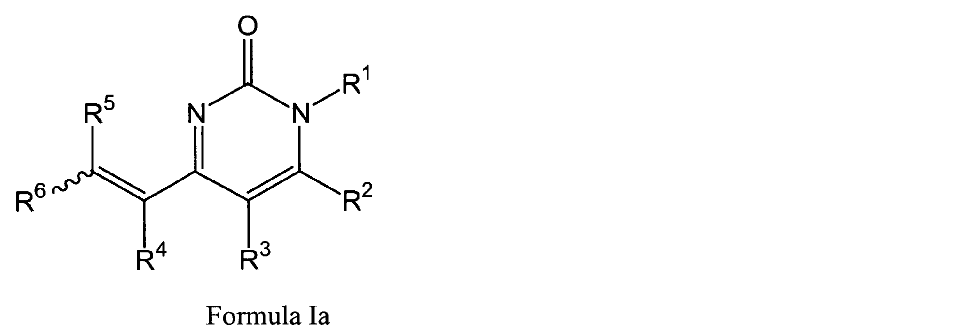 wherein:
R¹ represents hydrogen, C₁-C₆-alkyl, C₁-C₆-alkenyl, C₁-C₆-alkinyl, C₃-C₈-cycloalkyl, C₅-C-₁₅-aryl, heteroaryl, -COOR⁷ or -SO₂R⁷;
R² represents -C(R¹⁰)₃, -CR¹⁰(R^{10'})₂, -CR¹⁰(R^{10'})R^{10"}, -C₂(R¹⁰)₅, -CH₂-C(R¹⁰)_{3,} -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰, R^{10'}, R^{10"} represents F, Cl, Br or I;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
with the proviso that the following compounds are excluded:
4-(chlorodifluoromethyl )-6-[2-(4-hydroxy-3-methoxyphenyl )ethenyl]-1*H*-pyrimidine-2-one; 4-[2-(1,3-benzodioxol-5-yl)ethenyl]-6-chlorodifluoromethyl)-1*H*-pyrimidine-2-one; 4-[2-(3, 5-dichloro-2-methoxyphenyl)ethenyl]-6-(trifluoromethyl)-1*H*-pyrimidine-2-one; 4-[2-(6-chloro-1,3-benzodioxol-5-yl)-ethenyl]-6-(trifluoromethyl)-1*H*-pyrimidine-2-one; 4-[2-[4-(methylthio)-phenyl]ethenyl]-6-(trifluoromethyl)-1*H*-pyrimidine-2-one; 4-[2-(2,3-dichlorophenyl)ethenyl]-6-(trifluoromethyl)-1*H*-pyrimidine-2-one; 4-[2-[5-(2-methoxy-5-nitrophenyl)-2-furanyl]ethenyl]-6-(trifluoromethyl)-1*H*-pyrimidine-2-one; 4-[2-[5-(4-chloro-3-nitrophenyl)-2-furanyl]-ethenyl]-6-(trifluoromethyl)-1*H*-pyrimid ine-2-one; 4-[2-[5-(2-chloro-5-nitrophenyl)-2-furanyl]ethenyl]-6-(trifluoromethyl)-1*H*-pyrimidine-2-one; 4-[2-[5-(2-nitrophenyl)-2-furanyl]-ethenyl]-6-(trifluoromethyl)-1*H*-pyrimidine-2-one; 4-[2-(3-phenoxy-phenyl)-ethenyl]-6-(trifluoromethyl)-1*H*-pyrimidine-2-one; 4-[2-[4-[(4-fluorophenyl)-methoxy]phenyl]ethenyl]-6-(trifluoromethyl)-1*H*-pyrimidine-2-one; 3-[5-[2-[1,2-dihydro-2-oxo-6-(trifluoromethyl)-4-pyrimidinyl]ethenyl]-2-furanyl]-benzoic acid methyl ester; 4-[2-[3-ethoxy-4-(phenylmethoxy)phenyl]-ethenyl]-6-(trifluoromethyl)-1*H*-pyrimidine-2-one; 4-[2-[4-(1*H*-tetrazol-5-yl)-phenyl]ethenyl]-6-(trifluoromethyl)-1*H*-pyrimidine-2-one; 4-[2-[1,2-dihydro-2-oxo-6-(trifluoromethyl)-4-pyrimidinyl]-ethenyl]-benzonitrile; 5-chloro-2-[5-[2-[1,2-dihydro-2-oxo-6-(trifluoromethyl)-4-pyrimidinyl]ethenyl]-2-furanyl]-benzoic acid; 4-[2-[5-(3-chloro-4-fluorophenyl)-2-furanyl]ethenyl]-6-(trifluoromethyl)-1*H*-pyrimidine-2-one; 4-[2-(3-phenyl-1*H*-pyrazol-4-yl)ethenyl]-6-(trifluoromethyl)-1*H*-pyrimidine-2-one; 4-[2-[3,5-dichloro-2-[(2-fluorophenyl)-methoxy]phenylJethenyl]-6-(trifluoromethyl)-1*H*-pyrimidine-2-one; 4-[2-(2,4-difluorophenyl)ethenyl]-6-(trifluoromethyl)-1*H*-pyrimidine-2-one; 4-[2-(3-methyl-2-thienyl)ethenyl]-6-(trifluoromethyl)-1*H*-pyrimidine-2-one; 4-[2-[5-(4-nitrophenyl)-2-furanyl]ethenyl]-6-(trifluoromethyl)-1*H*-pyrimidine-2-one; 4-[2-[2-(benzoyloxy)phenyl]ethenyl]-6-(trifluoromethyl)-1*H*-pyrimidine-2-one; 4-[2-[4-(di-ethylamino)-2-methoxyphenyl]ethenyl]-6-(trifluoromethyl)-1*H*-pyrimidine-2-one; 4-[2-(4-bromo-3-nitrophenyl)ethenyl]-6-(trifluoromethyl)-1*H*-pyrimidine-2-one; 4-[2-[5-(3-chloro-4-methoxyphenyl)-2-furanyl]ethenyl]-6-(trifluoromethyl)-1*H*-pyrimidine-2-one; 4-[2-[1,2-dihydro-2-oxo-6-(trifluoromethyl)-4-pyrimidinyl]ethenyl]-benzoic acid methyl ester; 4-[2-(5-bromo-2-methoxyphenyl)ethenyl]-6-(trifluoromethyl)-1*H*-pyrimidine-2-one; 4-[2-(5-bromo-2-hydroxyphenyl)ethenyl]-6-(trifluoromethyl)-1*H*-pyrimidine-2-one; 4-[2-[5-(3-chloro-4-methylphenyl)-2-furanyl]ethenyl]-6-(trifluoro-methyl)-1*H*-pyrimidine-2-one; 4-[2-[4-(1,1-dimethylethyl)phenyl]ethenyl]-6-(tri-fluoro-methyl)-1*H*-pyrimidine-2-one; 4-[2-(3-bromo-4-hydroxy-5-methoxy-phenyl)-ethenyl]-6-(trifluoromethyl)-1*H*-pyrimidine-2-one; 4-[2-(2-hydroxy-3,5-diiodophenyl)ethenyl]-6-(trifluoromethyl)-1*H*-pyrimidine-2-one; 4-[2-(2-chlorophenyl)-ethenyl]-6-(trifluoromethyl)-1*H*-pyrimidine-2-one; 4-[2-(2,4-dimethoxyphenyl)-ethenyl]-6-(trifluoromethyl)-1*H*-pyrimidine-2-one; 4-[2-(6-bromo-1,3-benzodioxol-5-yl)ethenyl]-6-(trifluoromethyl)-1*H*-pyrimidine-2-one; 4-[2-(5-chloro-2-hydroxy-phenyl)ethenyl]-6-(trifluoromethyl)-1*H*-pyrimidine-2-one; 4-[2-(4-bromphenyl)-ethenyl]-6-(trifluoromethyl)-1*H*-pyrimidine-2-one; 4-[2-(3,4-dimethoxyphenyl)-ethenyl]-6-(trifluoromethyl)-1*H*-pyrimidine-2-one; 4-[2-[4-(diethylamino)-2-hydroxy-phenyl]ethenyl]-6-(trifluoromethyl)-1*H*-pyrimidine-2-one; 4-[2-(3,5-dibromo-2-hydroxyphenyl)-ethenyl]-6-(trifluoromethyl)-1*H*-pyrimidine-2-one; 5-[2-[1,2-dihydro-2-oxo-6-(trifluoromethyl)-4-pyrimidinyl]ethenyl]-1,3-dihydro-1,3-dimethyl-2*H*-benzimidazole-2-one; 5-[5-[2-[1,2-dihydro-2-oxo-6-(trifluoromethyl)-4-pyrimidinyl]-ethenyl]-2-furanyl]-2-hydroxy-benzoic acid; 4-[5-[2-[1,2-dihydro-2-oxo-6-(trifluoromethyl)-4-pyrimidinyl]ethenyl]-2-furanyl]-benzoic acid; 4-[2-[5-(4-bromophenyl)-2-furanyl]ethenyl]-6-(trifluoromethyl)-1*H*-pyrimidine-2-one; 4-[2-(4-chlorophenyl)-ethenyl]-6-(trifluoromethyl)-1*H*-pyrimidine-2-one; 4-[2-[5-(3-chlorophenyl)-2-furanyl]ethenyl]-6-(trifluoromethyl)-1*H*-pyrimidine-2-one; 4-[2-(3-bromophenyl)-ethenyl]-6-(trifluoromethyl)- 1*H*-pyrimidine-2-one; 4-[2-[4-(1 -pyrrolidinyl)phenyl]-ethenyl]-6-(trifluoromethyl)-1*H*-pyrimidine-2-one; 2-chloro-4-[5-[2-[1,2-dihydro-2-oxo-6-(trifluoromethyl)-4-pyrimidinyl]-ethenyl]-2-furanyl]-benzoic acid; 4-[2-[5-(2-chloro-4-nitrophenyl)-2-furanyl]-ethenyl]-6-(trifluoromethyl)-1*H*-pyrimidine-2-one; 4-[2-[5-(2-fluorophenyl)-2-furanyl]ethenyl]-6-(trifluoromethyl)-1*H*-pyrimidine-2-one; 4-chloro-2-[5-[2-[1,2-dihydro-2-oxo-6-(trifluoromethyl)-4-pyrimidinyl]ethenyl]-2-furanyl]-benzoic acid; 4-[2-(4-methoxyphenyl)ethenyl]-6-(trifluoromethyl)-1*H*-pyrimidine-2-one; 4-[2-(2-furanyl)ethenyl]-6-(trifluoromethyl)-1*H*-pyrimidine-2-one; 4-[2-[4-(dimethylamino)-3-nitrophenyl]ethenyl]-6-(trifluoromethyl)-1*H*-pyrimidine-2-one; 4-[2-(2-hydroxyphenyl)ethenyl]-6-(trifluoromethyl)-1*H*-pyrimidine-2-one; 4-[2-(4-hydroxyphenyl)ethenyl]-6-(trifluoromethyl)-1*H*-pyrimidine-2-one; 4-[2-[5-(2, 5-dimethyl-3-nitrophenyl)-2-furanyl]ethenyl]-6-(trifluoromethyl)-1*H*-pyrimidine-2-one; 4-[2-(3,5-dibromo-2-methoxyphenyl)-ethenyl]-6-(trifluoromethyl)-1*H*-pyrimidine-2-one; 4-[2-[5-(2,5-dimethyl-4-nitrophenyl)-2-furanyl]ethenyl]-6-(trifluoromethyl)-1*H*-pyrimidine-2-one; 4-[2-(3-ethoxy-4-hydroxyphenyl)ethenyl]-6-(trifluoromethyl)-1*H*-pyrimidine-2-one; 3-[5-[2-[1,2-dihydro-2-oxo-6-(trifluoromethyl)-4-pyrimidinyl]-ethenyl]-2-furanyl]-benzoic acid; 4-[2-(2,4-dichlorophenyl)ethenyl]-6-(trifluoromethyl)-1*H*-pyrimidine-2-one; 4,4'-(1,4-phenylenedi-2,1-ethenediyl)bis[6-(trifluoro-methyl)-1*H*-pyrimidine-2-one; 4-[2-[5-(2,4-dimethyl-5-nitrophenyl)-2-furanyl]-ethenyl]-6-(trifluoromethyl)-1*H*-pyrimidine-2-one; 4-[2-[5-(2-methoxy-4-nitro-phenyl)-2-furanyl]ethenyl]-6-(trifluoromethyl)-1*H*-pyrimidine-2-one; 4-[2-[5-(2,4-dichlorophenyl)-2-furanyl]ethenyl]-6-(trifluoromethyl)-1*H*-pyrimidine-2-one; 4-[2-[3,5-dibromo-2-[(2,4-dichlorophenyl)methoxy]phenyl]ethenyl]-6-(trifluoromethyl)-1*H*-pyrimidine-2-one; 4-[2-[5-(3-nitrophenyl)-2-furanyl]-ethenyl]-6-(trifluoromethyl)-1*H*-pyrimidine-2-one; 4-[2-(2,4-dichloro-5-nitrophenyl)ethenyl]-6-(trifluoromethyl)-1*H*-pyrimidine-2-one; 4-[2-[4-(dimethylamino)phenyl]ethenyl]-6-(trifluoromethyl)-1*H*-pyrimidine-2-one; 4-[2-(1-naphthalenyl)ethenyl]-6-(trifluoromethyl)-1*H*-pyrimidine-2-one; 4-[2-(3-fluorophenyl )ethenyl]-6-(trifluoromethyl)-1*H*-pyrimidine-2-one; 4-(trifluoromethyl)-6-[2-(2,4,5-trimethylphenyl)ethenyl]-1*H*-pyrimidine-2-one; 4-[2-[4-(4-morpholinyl)phenyl]ethenyl]-6-(trifluoromethyl)-1*H*-pyrimidine-2-one; 4-[2-(9-ethyl-9H-carbazol-3-yl)ethenyl]-6-(trifluoromethyl)-1*H*-pyrimidine-2-one; 4-[2-[5-(4-methoxy-2-nitrophenyl)-2-furanyl]ethenyl]-6-(trifluoromethyl)-1*H*-pyrimidine-2-one; [2-[2-[1,2-dihydro-2-oxo-6-(trifluoromethyl)-4-pyrimidinyl]-ethenyl]-6-methoxyphenoxy]-acetic acid ethyl ester; 4-[2-(1,3-benzodioxol-5-yl)ethenyl]-6-(trifluoromethyl)-1*H*-pyrimidine-2-one; 4-[2-[1 -[(2-fluorophenyl)methyl]-1*H*-indol-3-yl]ethenyl]-6-(trifluoromethyl)-1*H*-pyrimidine-2-one; [4-[2-[1,2-dihydro-2-oxo-6-(trifluoromethyl)-4-pyrimidinyl]ethenyl]-2-methoxyphenoxy]-acetic acid ethyl ester; 4-[2-[5-(5-chloro-2-methoxyphenyl)-2-furanyl]ethenyl]-6-(trifluoro-methyl)-1*H*-pyrimidine-2-one; 4-[2-(2,3-dimethoxyphenyl)ethenyl]-6-(trifluoro-methyl)-1*H*-pyrimidine-2-one; 8-[4-[2-[1,2-dihydro-2-oxo-6-(trifluoromethyl)-4-pyrimidinyl]ethenyl]-2-methoxyphenoxy]-3,7-dihydro-1,3,7-trimethyl-1*H*-purine-2,6-dione; 4-[2-[5-( 1,3-d ihydro-1-oxo-5-isobenzofuranyl)-2-furanyl]ethenyl]-6-(trifluoromethyl)-1*H*-pyrimidine-2-one; 4-[2-[1 -[(3,4-dichlorophenyl)methyl]-3-phenyl-1*H*-pyrazol-4-yl]ethenyl]-6-(trifluoromethyl)-1*H*-pyrimidine-2-one; 4-[2-[5-(2,3-dichlorophenyl)-2-furanyl]ethenyl]-6-(trifluoromethyl)-1*H*-pyrimidine-2-one; 4-[2-[4-[(2,4-dichlorophenyl)methoxy]-3-methoxyphenyl]ethenyl]-6-(trifluoromethyl)-1*H*-pyrimidine-2-one; 4-[2-(4-hydroxy-3-methoxyphenyl)ethenyl]-6-(trifluoromethyl)-1*H*-pyrimidine-2-one; 4-[2-(2-chloro-5-nitrophenyl)ethenyl]-6-(trifluoromethyl)-1*H*-pyrimidine-2-one; 4-[2-[5-(4-fluorophenyl)-2-furanyl]-ethenyl]-6-(trifluoromethyl)-1*H*-pyrimidine-2-one; 4-[2-[5-(3,5-dichlorophenyl)-2-furanyl]ethenyl]-6-(trifluoro-methyl)-1*H*-pyrimidine-2-one; 4-(trifluoromethyl)-6-[2-[5-[3-(trifluoromethyl)phenyl]-2-furanyl]ethenyl]-1*H*-pyrimidine-2-one; 2-chloro-5-[5-[2-[1,2-dihydro-2-oxo-6-(tri-fluoromethyl)-4-pyrimidinyl]ethenyl]-2-furanyl]-benzoic acid; 4-[2-(7-methoxy-2-oxo-1,3-benzoxathiol-5-yl)ethenyl]-6-(trifluoromethyl)-1*H*-pyrimidine-2-one; 4-[2-(3-nitrophenyl)ethenyl]-6-(trifluoromethyl)-1*H*-pyrimidine-2-one; 4-[2-(4-hydroxy-3, 5-dimethoxyphenyl)ethenyl]-6-(trifluoromethyl)-1*H*-pyrimidine-2-one; 4-[2-(3-hydroxy-phenyl)ethenyl]-6-(trifluoromethyl)-1*H*-pyrimidine-2-one; 4-(trifluoromethyl)-6-[2-(2,4,5-trimethoxyphenyl)ethenyl]-1*H*-pyrimidine-2-one; 4-[2-(3,5-diiodo-2-methoxy-phenyl)ethenyl]-6-(trifluoromethyl)-1*H*-pyrimidine-2-one; 4-[2-(3-ethoxy-4-methoxy-phenyl)ethenyl]-6-(trifluoromethyl)-1*H*-pyrimidine-2-one; 4-(trifluoromethyl)-6-[2-(3,4,5-trimethoxyphenyl)ethenyl]-1*H*-pyrimidine-2-one; 4-[2-(3-bromo-4-methoxy-phenyl)-ethenyl]-6-(trifluoromethyl)-1*H*-pyrimidine-2-one; 2-[5-[2-[1,2-dihydro-2-oxo-6-(trifluoromethyl)-4-pyrimidinyl]ethenyl]-2-furanyl]-benzoic acid; 4-[2-(6-nitro-1,3-benzodioxol-5-yl )ethenyl]-6-(trifluoromethyl)-1*H*-pyrimidine-2-one; 4-(trifluoro-methyl)-6-[2-[2-(trifluoromethyl)phenyl]ethenyl]-1*H*-pyrimidine-2-one; 4-[2-(2-hydroxy-1 -naphthalenyl)ethenyl]-6-(trifluoromethyl )-1*H*-pyrimidine-2-one; 4-[2-[1 -(4-nitrophenyl)-1*H*-pyrrol-2-yl]ethenyl]-6-(trifluoromethyl)-1*H*-pyrimidine-2-one;
the C₁-C₆-alkyl, C₃-C₈-cycloalkyl, C₅-C₁₅-aryl, and heteroaryl group can optionally be substituted by one or more substituents R⁸;
R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyi, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;
R⁹ represents hydrogen, C₁-C₆-hydroxyalkyl, C₁-C₆-alkyl, C₁-C₆-aminoalkyl, C₅-C₁₅-aryl or heteroaryl.

4. Compound selected from the following group comprising:
4-{2-[4-(2-Chloro-6-fluoro-benzyloxy)-3,5-dimethoxy-phenyl]-vinyl}-6-trifluoromethyl-1H-pyrimidine-2-one; 6-Trifluoromethyl-4-[2-(4-trifluoromethylphenyl)vinyl]-1H-pyrimidine-2-one; 4-[2-(2-Hyd roxy-4-methoxy-phenyl)-vinyl]-6-trifluoromethyl-1H-pyrimidine-2-one; 4-[2-(5-Bromo-2-hydroxy-3-methoxy-phenyl)vinyl]-6-trifluoro-methyl-1H-pyrimidine-2-one; 4-[2-(2,6-Dichloro-phenyl)vinyl]-6-trifluoromethyl-1H-pyrimidine-2-one; 5-(4-Chlorophenyl)-2-[2-(2-oxo-6-trifluoromethyl-1,2-dihydro-pyrimidine-4-yl)-vinyl]-furan-3-carboxylic acid ethyl ester; 4-[2-(2-Oxo-6-trifluoromethyl-1,2-dihydro-pyrimidine-4-yl)-vinyl]-benzoic acid; 6-Trifluoromethyl-4-{2-[5-(2-trifluoromethyl-phenyl)-furan-2-yl]-vinyl}-1H-pyrimidine-2-one; 5-{1-[2-(2-Oxo-6-trifluoromethyl-1,2-dihydro-pyrimidine-4-yl)-vinyl]-naphthalene-2-yloxymethyl}-4,5-dihydro-isoxazole-3-carboxylic acid ethyl ester; 4-[2-(2-Allyloxy-naphthalene-1 -yl)-vinyl]-6-trifluoromethyl-1H-pyrimidine-2-one; 4-[2-(3-Phenyl-benzo[c]isoxazol-5-yl)-vinyl]-6-trifluoromethyl-1H-pyrimidine-2-one; 4-{2-[2-(2-Oxo-6-trifluoromethyl-1,2-dihydro-pyrimidine-4-yl )-vinyl]-phenyl}-piperazine-1-carboxylic acid ethyl ester; 6-Trifluoromethyl-4-(2-{2-[4-(5-trifluoromethyl-pyridine-2-yl)-piperazine-1-yl]-phenyl}-vinyl)-1H-pyrimidine-2-one; 4-{2-[2-(4-Benzyl-piperazine-1-yl)-phenyl]-vinyl}-6-trifluoromethyl-1H-pyrimidine-2-one; 2-Chloro-5-{5-[2-(2-oxo-6-trifluoromethyl-1,2-dihydro-pyrimidine-4-yl)-vinyl]-furan-2-yl}-benzoic acid; 4-{2-[5-(3-Methyl-4-nitro-phenyl)-furan-2-yl]-vinyl}-6-trifluoromethyl-1H-pyrimidine-2-one; 6-Methyl-4-[2-(4-tri-fluoromethyl-phenyl)-vinyl]-1H-pyrimidine-2-one; 4,6-Bis-[2-(5-bromo-2-hydroxy-3-methoxyphenyl)-vinyl]-pyrimidine-2-ol; 4-{2-[5-(2-Chloro-phenyl)-furan-2-yl]-vinyl}-6-trifluoromethyl-1H-pyrimidine-2-one; 4-{2-[5-(4-Chloro-phenyl)-furan-2-yl]-vinyl}-6-trifluoromethyl-1H-pyrimidine-2-one; 4-[2-(5-Hydroxymethyl-furan-2-yl)-vinyl]-6-tri-fluoromethyl-1H-pyrimidine-2-one; 4,6-Bis-[2-(2-tri-fluoromethyl-phenyl)-vinyl]-pyrimidine-2-ol; 4-[2-(2,6-Dichlorophenyl)-vinyl]-6-methylpyrimidine-2-ol; 4-[2-(5-Methyl-thiophen-2-yl)-vinyl]-6-trifluoromethyl-1H-pyrimidine-2-one; 4-(2-Thiophen-2-yl-vinyl)-6-trifluoro-methyl-1H-pyrimidine-2-one; 4-[2-(4-Bromo-5-tert-butylthiophen-2-yl)-vinyl]-6-trifluoromethyl-1H-pyrimidine-2-one; 4-{2-[3-(2,4-Dichlorobenzyloxy)-naphthalene-2-yl]-vinyl}-6-trifluoromethyl-1H-pyrimidine-2-one; 4-[2-(2-Hydroxynaphthalene-1-yl)-vinyl]-6-methylpyrimidine-2-ol; 4-[2-(3,5-Di-bromo-2-hydroxyphenyl)-vinyl]-6-methylpyrimidine-2-ol; 4-{2-[5-(2-Chloro-5-trifluoromethyl-phenyl)-furan-2-yl]-vinyl}-6-trifluoromethyl-1H-pyrimidine-2-one; 4-[2-(5-Bromofuran-2-yl)-vinyl]-6-trifluoromethyl-1H-pyrimidine-2-one; 4-(2-Benzofuran-2-yl-vinyl)-6-trifluoromethyl-1H-pyrimidine-2-one; 4-[2-(5-Bromothiophen-2-yl)-vinyl]-6-trifluoromethyl-1H-pyrimidine-2-one; 2-[2-(2-Oxo-6-trifluoromethyl-1,2-dihydro-pyrimidine-4-yl)-vinyl]-thiophene-3-carboxylic acid; 4-[2-(4-Trifluoromethyl-phenyl)-vinyl]-pyrimidine-2-ol; 4-[2-(2-Trifluoromethylphenyl)-vinyl]-pyrimidine-2-ol; 4-[2-(3,5-Dibromo-2-hydroxy-phenyl)-vinyl]-pyrimidine-2-ol; 4-[2-(2,6-Dichlorophenyl)-vinyl]-pyrimidine-2-ol; 4-[2-(2-Hydroxy-naphthalene-1-yl)-vinyl]-pyrimidine-2-ol; 4-[2-(5-Bromo-2-hydroxy-3-methoxyphenyl)-vinyl]-6-methyl-pyrimidine-2-ol; 4-[2-(2-Hydroxynaphthalene-1-yl)-1-methylvinyl]-6-trifluoromethyl-1H-pyrimidine-2-one; 4-[2-(2-Hydroxynaphthalene-1 -yl)-vinyl]-6-phenyl-1H-pyrimidine-2-one; 4-[1-Methyl-2-(4-trifluoromethyl-phenyl)-vinyl]-6-trifluoromethyl-1H-pyrimidine-2-one; 6-(Chlorodifluoromethyl)-4-[2-(4-trifluoromethyl-phenyl)-vinyl]-1H-pyrimidine-2-one; 6-Phenyl-4-[2-(4-trifluoromethyl-phenyl)-vinyl]-1H-pyrimidine-2-one; 6-(Chlorodifluoro-methyl)-4-[2-(3,5-dibromo-2-hydroxyphenyl)-vinyl]-1H-pyrimidine-2-one; 4-[2-(3,5-Dibromo-2-hydroxyphenyl)-vinyl]-6-phenyl-1H-pyrimidine-2-one; 4-[2-(2,6-Dichloro-phenyl)-1 -methylvinyl]-6-trifluoro-methyl-1H-pyrimidine-2-one; 6-(Chloro-difluoro-methyl)-4-[2-(2,6-dichloro-phenyl)-vinyl]-1H-pyrimidine-2-one; 4-[2-(2,6-Dichlorophenyl)-vinyl]-6-phenyl-1H-pyrimidine-2-one; 4-[2-(3,5-Dibromo-2-hydroxyphenyl)-1 -methyl-vinyl]-6-trifluoromethyl- 1H-pyrimidine-2-one; 4-[1-Methyl-2-(2-tri-fluoromethylphenyl)-vinyl]-6-trifluoromethyl-1H-pyrimidine-2-one; 6-Phenyl-4-[2-(2-trifluoromethyl-phenyl)-vinyl]-1H-pyrimidine-2-one.

5. A process for the preparation of a compound of formula (I) which comprises the step of reacting a pyrimidone of formula (II) with a carbonyl compound of formula (III).

6. A process for the preparation of a compound of formula (la) which comprises the step of reacting a pyrimidone of formula (IIa) with a carbonyl compound of formula (III).

7. A compound for the use as pharmaceutically active agent having the general formula (I) and pharmaceutically acceptable salts thereof, 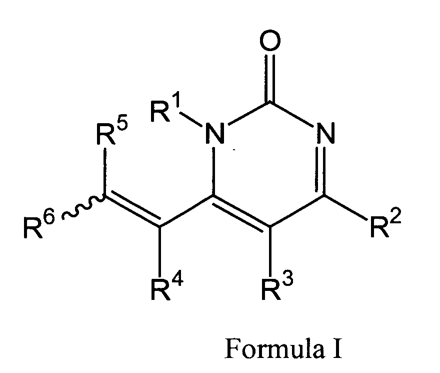 wherein:
R¹ represents C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷, -SO₂R⁷, ₋C(R¹⁰)₃, -CR¹⁰(R¹⁰')₂, -CR¹⁰(R^{10'})R^{10"}, -C₂(R¹⁰)_{5,} -CH₂-C(R¹⁰)_{3,} -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰, R^{10'}, R^{10"} represents F, Cl, Br or I;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
the C₁-C₆-alkyl, C₃-C₈-cycloalkyl, C₅-C₁₅-aryl, heteroaryl group can optionally be substituted by one or more substituents R⁸;
R⁸ represent independently of each other hydrogen, -COOR⁹, -CONHR⁹, -CO-R⁹, -SO₂NHR⁹, -SO₂N(R⁹)₂, -NO₂, -F, -CI, -Br, -I, -CN, -OH, -SH, -NR⁹-CO-(C₁-C₆)-haloalkyl, -NR⁹-SO₂-(C₁-C₆)-haloalkyl, C₁-C₆-alkyl, C₁-C₆-aminoalkyl, C₁-C₆-alkoxy, C₁-C₆-alkylthio, C₁-C₆-hydroxyalkyl-amino, C₁-C₆-haloalkyloxy, C₅-C₁₅-aryl or heteroaryl;
R⁹ represents hydrogen, C₁-C₆-hydroxyalkyl, C₁-C₆-alkyl, C₁-C₆- aminoalkyl, C₅-C₁₅-aryl or heteroaryl.

8. A compound for the use as pharmaceutically active agent having the general formula (la) and pharmaceutically acceptable salts thereof, 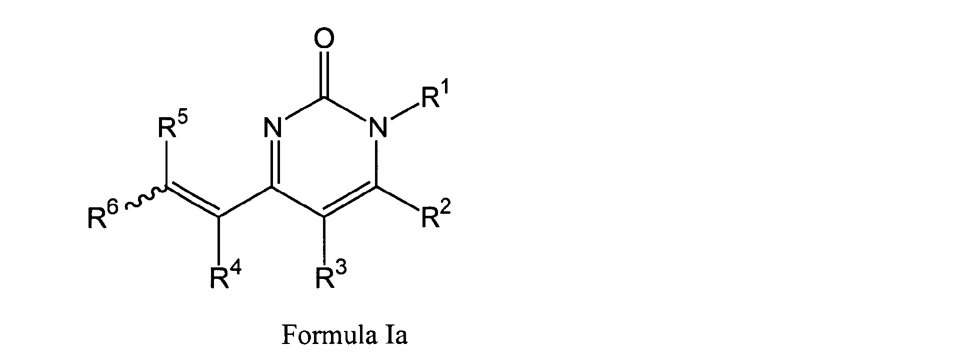 wherein:
R¹ represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷ or -SO₂R⁷;
R² represents hydrogen, C₁-C₆-alkyl, C₁-C₆-alkenyl, C₁-C₆-alkinyl, C₃-C₈-cycloalkyl, C₅-C₁₅-aryl, heteroaryl, -COOR⁷, -SO₂R⁷, -C(R¹⁰)₃, -CR¹⁰(R^{10'})₂, -CR¹⁰(R^{10'})R^{10"}, -C₂(R¹⁰)_{5,} -CH₂-C(R¹⁰)_{3,} -CH₂-CR¹⁰(R^{10'})₂, -CH₂-CR¹⁰(R^{10'})R^{10"}, -C₃(R¹⁰)₇ or -C₂H₄-C(R¹⁰)₃;
R¹⁰,R^{10'},R^{10"} represents F, Cl, Br or I;
R³, R⁴, R⁵ and R⁷ represent independently of each other hydrogen, C₁-C₆-alkyl, C₁-C₆-alkenyl, C₁-C₆-alkinyl, C₃-C₈-cycloalkyl, C₅-C₁₅-aryl or heteroaryl;
R⁶ represents C₁-C₆-alkyl, C₁-C₆-alkenyl, C₁-C₆-alkinyl, C₃-C₈-cycloalkyl, C₅-C₁₅-aryl or heteroaryl;
with the proviso that when R⁵ or R⁶ are pyridine or phenyl and R² is methyl; and 4-(2,2-diphenylethenyl)-6-methyl-1H-pyrimdin-2-one are excluded, for the use as pharmaceutically active agent;
the C₁-C₆-alkyl, C₃-C₈-cycloalkyl, C₅-C₁₅-aryl, and heteroaryl group can optionally be substituted by one or more substituents R⁸;
R⁸ represents independently of each other hydrogen, -COOR⁹, -CONHR⁹, -F, -Cl, -Br, -I, -CO-R⁹, -SO₂NHR⁹, -SO₂N(R⁹)₂, -NR⁹-CO-(C₁-C₆)-haloalkyl, -NO₂, -NR⁹-SO₂-(C₁-C₆)-haloalkyl, -CN, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-haloalkyloxy, C₅-C₁₅-aryl or heteroaryl;
R⁹ represents hydrogen, C₁-C₆-hydroxyalkyl, C₁-C₆-alkyl, C₁-C₆-aminoalkyl, C₅-C₁₅-aryl or heteroaryl.

9. The compound of claim 8, wherein R¹ is H, R² is CF₃ and R⁶ is furyl, which can optionally be substituted by one or more substituents R⁸, R⁸ is as defined above.

10. The compound of claim 8, wherein R¹ is H, R² is CF₃ and R⁶ is naphthyl, which can optionally be substituted by one or more substituents R⁸, R⁸ is as defined above.

11. The compound of claim 8, wherein R¹ is H, R² is methyl and R⁶ is phenyl, which can optionally be substituted by one or more substituents R⁸, R⁸ is as defined above.

12. The compound according to any one of claims 7 - 11 wherein the compound is selected from the group comprising:
4-[2-(2-Bromo-5-methoxy-4-prop-2-ynyloxy-phenyl)-vinyl]-6-trifluoromethyl-1*H*-pyrimidine-2-one; 4-[2-(3-Chloro-4, 5-diethoxyphenyl)-vinyl]-6-trifluoromethyl-1*H*-pyrimidine-2-one; 4-[2-(2,3-Dimethoxyphenyl)-vinyl]-6-trifluoromethyl-1*H*-pyrimidine-2-one; 4-[2-(2-Chloro-4-hydroxy-5-methoxyphenyl)-vinyl]-6-trifluoromethyl-1*H*-pyrimidine-2-one; 4-{2-[4-(4-Fluorobenzyloxy)-phenyl]-vinyl}-6-trifluoromethyl-1*H*-pyrimidine-2-one;4-[2-(2-Hydroxynaphtalen-1-yl)-vinyl]-6-trifluoromethyl-1*H*-pyrimidine-2-one; 4-{2-[4-(2-Fluorobenzyloxy)-3-methoxy-phenyl]-vinyl}-6-trifluoromethyl-1*H*-pyrimidine-2-one; 4-[2-(4-Benzyloxy-3-ethoxy-phenyl)-vinyl]-6-trifluoromethyl-1*H*-pyrimidine-2-one; 4-[2-(3-Chloro-4,5-dimethoxy-phenyl)-vinyl]-6-trifluoromethyl-1*H*-pyrimidine-2-one; 4-[2-(3-Hydroxyphenyl)-vinyl]-6-trifluoromethyl-1*H*-pyrimidine-2-one; 4-{2-[4-(2-Chloro-6-fluoro-benzyloxy)-3, 5-dimethoxyphenyl]-vinyl}-6-trifluoromethyl-1*H*-pyrimidine-2-one; 4-{2-[4-(2-Fluorobenzyl-oxy)-phenyl]-vinyl}-6-trifluoromethyl-1*H*-pyrimidine-2-one; 3-{5-[[2-(2-Oxo-6-trifluoromethyl-1,2-dihydropyrimidine-4-yl)-vinyl]-furan-2-yl}-benzoic acid methyl ester; 4-{2-[5-(2,3-Dichlorophenyl)-furan-2-yl]-vinyl}-6-trifluoromethyl-1*H*-pyrimidine-2-one; 4-[2-(2-Chloro-5-nitrophenyl)-vinyl]-6-trifluoromethyl-1*H*-pyrimidine-2-one; 4-{2-[5-(2,5-Dimethyl-4-nitrophenyl)-furan-2-yl]-vinyl}-6-trifluoromethyl-1*H*-pyrimidine-2-one; 4-{2-[5-(2-Chloro-4-nitrophenyl)-furan-2-yl]-vinyl}-6-trifluoromethyl-1*H*-pyrimidine-2-one; 4-{2-[5-(1 -Oxo-1,3-dihyd ro-isobenzofuran-5-yl)-furan-2-yl]-vinyl}-6-trifluoro-methyl-1*H*-pyrimidine-2-one; 4-[2-(4-Chlorophenyl)-vinyl]-6-trifluoromethyl-1*H*-pyrimidine-2-one; 4-[2-(2,4-Difluorophenyl)-vinyl]-6-trifluoromethyl-1*H*-pyrimidine-2-one; 4-[2-(3-Ethoxy-4-hydroxyphenyl)-vinyl]-6-trifluoromethyl-1*H*-pyrimidine-2-one; 4-[2-(4-Di-methylamino-3-nitrophenyl)-vinyl]-6-trifluoromethyl-1*H*-pyrimidine-2-one; 4-[2-(4-Methoxy-phenyl)-vinyl]-6-trifluoromethyl-1*H*-pyrimidine-2-one; 4-[2-(4-Hydroxy-5-methoxyphenyl)-vinyl]-6-trifluoromethyl-1*H*-pyrimidine-2-one; Carbonic acid ethyl ester 2 methoxy-4-[2-(2-oxo-6-trifluormethyl-1,2-dihydropyrimidine-4-yl)-vinyl]-phenyl ester; 4-[2-(4-Hydroxy-3,5-dimethoxy-phenyl)-vinyl]-6-trifluoromethyl-1*H*-pyrimidine-2-one; 4-[2-(4-Hydroxyphenyl)-vinyl]-6-trifluoromethyl-1*H*-pyrimid ine-2-one; 4-[2-(2-Hydroxy-4-methoxy-phenyl)-vinyl]-6-trifluoromethyl-1*H*-pyrimidine-2-one; 4-[2-(3-Bromo-6-hydroxy-5-methoxyphenyl)-vinyl]-6-trifluoromethyl-1*H*-pyrimidine-2-one; 4-[2-(3,5-Dibromo-2-hydroxyphenyl)-vinyl]-6-trifluoromethyl-1*H*-pyrimidine-2-one; 4-[2-(2,6-Dichlorophenyl)-vinyl]-6-trifluoromethyl-1*H*-pyrimidine-2-one; 5-(4-Chlorophenyl)-2-[2-(2-oxo-6-trifluoromethyl-1,2-dihydro-pyrimidine-4-yl)-vinyl]-furan-3-carboxylic acid ethyl ester; 4-[2-(2-Trifluoromethylphenyl)-vinyl]-6-trifluoromethyl-1*H*-pyrimidine-2-one; 4-[2-(2-Oxo-6-trifluoromethyl-1,2-dihydro-pyrimidine-4-yl)-vinyl]-benzoic acid; 4-{2-[5-(2-Trifluoromehtylphenyl)-furan-2-yl]-vinyl}-6-trifluoromethyl-1*H*-pyrimidine-2-one; 5-{1 -[2-(2-Oxo-6-trifluoromethyl-1,2-dihydropyrimidine-4-yl)-vinyl]-naphtalene-2-yloxymethyl}-4,5-dihydro-isoxazole-3-carboxylic acid ethyl ester; 4-[2-(2-Allyloxynaphtalene-1 -yl)-vinyl]-6-trifluoromethyl-1*H*-pyrimidine-2-one; 4-[2-(3-Phenylbenzo[c]isoxazol-5-yl)-vinyl]-6-trifluoromethyl-1*H*-pyrimidine-2-one; 4-{2-[2-(2-Oxo-6-trifluoromethyl-1,2-dihydropyrimidine-4-yl)-vinyl]-phenyl}-piperazine-1-carboxylic acid ethyl ester; 6-Trifluoromethyl-4-2-{2-[4-(5-trifluoromethyl-pyridine-2-yl)-piperazine-1-yl]-phenyl}-vinyl-1*H*-pyrimidine-2-one; 4-{2-[2-(4-Benzyl-piperazine-1-yl)-phenyl]-vinyl}-6-trifluoromethyl-1*H*-pyrimidine-2-one; 2-Methyl-5-{5-[2-(2-Oxo-6-trifouoromethyl-1,2-dihydropyrimidine-4-yl)-vinyl]-furan-2-yl}-benzoic acid; 4-{2-[5-(3-Chloro-4-methylphenyl)-furan-2-yl]-vinyl}-6-trifluoromethyl-1*H*-pyrimidine-2-one; 4-[2-(9-Ethyl-9H-carbazole-3-yl)-vinyl]-6-trifluoromethyl-1*H*-pyrimidine-2-one; 4-{2-[5-(4-Bromophenyl)-furan-2-yl]-vinyl}-6-trifluoromethyl-1*H*-pyrimidine-2-one; 4-(2-Benzo[1,3]dioxol-5-yl-vinyl)-6-trifluoro-methyl-1*H*-pyrimidine-2-one; 4-[2-(3,4, 5-Trimethoxyphenyl)-vinyl]-6-trifluoromethyl-1*H*-pyrimidine-2-one; 4-[2-(4-Chlorobenzo[1,3]dioxol-5-yl)-vinyl]-6-trifluoromethyl-1*H*-pyrimidine-2-one; 4-{2-[5-(3-Chlorophenyl)-furan-2-yl]-vinyl}-6-trifluoromethyl-1*H*-pyrimidine-2-one; 4-[2-(4-Pyrrolidin-1-yl-phenyl)-vinyl]-6-trifluoromethyl-1*H*-pyrimidine-2-one; 4-{2-[5-(4-Methoxy-2-nitrophenyl)-furan-2-yl]-vinyl}-6-trifluoro-methyl-1*H*-pyrimidine-2-one; 4-{2-[5-(3-Methyl-4-nitrophenyl)-furan-2-yl]-vinyl}-6-trifluoromethyl-1*H*-pyrimidine-2-one; 4-[2-(4-Trifluoromethylphenyl)-vinyl]-6-trifluoromethyl-1*H*-pyrimid ine-2-one; 4-(2-Furan-2-yl )-vinyl-6-trifluoromethyl-1*H*-pyrimidine-2-one; 4-{2-[5-(2-Chlorophenyl)-furan-2-yl]-vinyl}-6-trifluoro-methyl-1*H*-pyrimidine-2-one; 4-{2-[5-(3-Nitrophenyl)-furan-2-yl]-vinyl}-6-tri-fluoromethyl-1*H*-pyrimidine-2-one; 4-{2-[5-(4-Chlorophenyl)-furan-2-yl]-vinyl}-6-trifluoromethyl-1*H*-pyrimidine-2-one; 4-{2-[5-(2-Nitrophenyl)-furan-2-yl]-vinyl}-6-trifluoromethyl-1*H*-pyrimidine-2-one; 4-[2-(5-Hydroxymethyl-furan-2-yl)-vinyl]-6-trifluoromethyl-1*H*-pyrimidine-2-one; 4-[2-(5-Methylthiophen-2-yl)-vinyl]-6-trifluoromethyl-1*H*-pyrimidine-2-one; 4-(2-Thiophen-2-yl)-vinyl-6-tri-fluoromethyl-1H-pyrimidine-2-one; 4-[2-(4-Bromo-5-tert-butylthiophen-2-yl)-vinyl]-6-trifluoromethyl-1*H*-pyrimidine-2-one; 4-{2-[3-(2,4-Dichlorobenzyloxy)-naphtalene-2-yl]-vinyl}-6-trifluoromethyl-1*H*-pyrimidine-2-one; 4-{2-[5-(2-Chloro-5-trifluoromethyl-phenyl)-furan-2-yl]-vinyl}-6-trifluoromethyl-1*H*-pyrimidine-2-one; 4-[2-(5-Bromofuran-2-yl)-vinyl]-6-trifluoromethyl-1*H*-pyrimidine-2-one; 4-(2-Benzofuran-2-yl-vinyl)-6-trifluoromethyl-1*H*-pyrimidine-2-one; 4-[2-(5-Bromothiophen-2-yl)-vinyl]-6-trifluoromethyl-1*H*-pyrimidine-2-one; 4-[2-(5-Methylthiophen-2-yl)-vinyl]-6-trifluoromethyl-1*H*-pyrimidine-2-one; 2-[2-(2-oxo-6-trifluoromethyl-1,2-dihydropyrimidine4-yl)-vinyl]-thiophene-3-carboxylic acid; 4-[2-(3-Hydroxynaphtalene-2-yl)-1-methylvinyl]-6-trifluoromethyl-1*H*-pyrimidine-2-one; 4-[2-(3-Hydroxynaphtalene-2-yl)-vinyl]-6-phenyl-1*H*-pyrimidine-2-one; 4-[2-(4-Trifluoromethylphenyl)-1-methyl-vinyl]-6-trifluoromethyl-1*H*-pyrimidine-2-one; 6-(Chlorodifluoromethyl)-4-[2-(4-tri-fluoromethylphenyl)-vinyl]-1*H*-pyrimidine-2-one; 4-[2-(4-Trifluoromethyl-phenyl)-vinyl]-6-phenyl-1*H*-pyrimidine-2-one; 6-(Chlorodifluoromethyl)-4-[2-(3,5-Dibromo-6-hydroxyphenyl)-vinyl]-1*H*-pyrimidine-2-one; 4-[2-(3,5-Di-bromo-6-hydroxyphenyl)-vinyl]-6-phenyl-1*H*-pyrimidine-2-one; 4-[2-(2,6-Di-chlorophenyl)-1 -methylvinyl]-6-trifluoromethyl-1*H*-pyrimidine-2-one; 6-(Chlorodifluoromethyl)-4-[2-(2,6-Dichloro-phenyl)-vinyl]-1*H*-pyrimidine-2-one; 4-[2-(2,6-Dichlorophenyl)-vinyl]-6-phenyl-1*H*-pyrimidine-2-one; 4-[2-(3,5-Dibromo-6-hydroxyphenyl)-1-methyl-vinyl]-6-trifluoro-methyl-1*H*-pyrimidine-2-one; 4-[2-(2-Trifluoromethylphenyl)-1-methylvinyl]-6-trifluoromethyl-1*H*-pyrimidine-2-one; 6-(Chlorodifluoro-methyl)-4-[2-(2-trifluoro-methylphenyl)-vinyl]-1*H*-pyrimidine-2-one; 4-[2-(2-Trifluoromethylphenyl)-vinyl]-6-phenyl-1*H*-pyrimidine-2-one; 4-[2-(2-Hydroxynapthalene-1-yl)-vinyl]-pyrimidine-2-one; 4,6-Bis-[2-(3,5-dibromo-2-hydroxyphenyl)-vinyl]-pyrimidine-2-one; 6-[2-(6-Nitrobenzo[1,3]dioxol-5-yl)-vinyl]-4-trifluoromethyl-1*H*-pyrimidine-2-one; 6-Trifluoro-methyl-4-[3-(4-trifluoromethylphenyl)-vinyl]-1*H*-pyrimidine-2-one; 4-[2-(3,5-Dibromo-2-hydroxy-phenyl)-vinyl]-pyrimidine-2-one; 4-[2-(3-Hydroxynaphthalene-2-yl)-vinyl]-pyrimidine-2-one; 4-[2-(3,5-Dibromo-2-hydroxyphenyl)-vinyl]-6-methyl-pyrimidine-2-one; 4,6-Bis-[2-(5-bromo-2-hydroxy-3-methoxyphenyl)-vinyl]-pyrimidine-2-one; 4-{2-[4-(2,4-Dichlorobenzyloxy)-3-methoxyphenyl]-vinyl}-6-tri-fluoromethyl-1*H*-pyrimidine-2-one; 4,6-Bis-[2-(4-isopropylphenyl)-vinyl]-pyrimidine-2-one; 4-[2-(5-methylthiophen-2-yl]-vinyl]-6-trifluoromethyl-1*H*-pyrimidine-2-one; 6-Trifluoromethyl-4-[2-(2-trifluoromethylphenyl)-vinyl]-1*H*-pyrimidine-2-one; 4,6-Bis-[2-(2-trifluoro-methylphenyl)-vinyl]-pyrimidine-2-one; 4-[2-(2,6-Dichlorophenyl)-vinyl]-6-methylpyrimidine-2-one; 4-[2-(2,6-Dichlorophenyl)-vinyl]-pyrimidine-2-one; 4-[2-(2-Trifluoromethylphenyl)-vinyl]-pyrimidine-2-one; 4-[2-(5-Bromo-2-hydroxy-3-methoxyphenyl)-vinyl]-6-methyl-pyrimidine-2-one and salts of these compounds.

13. The use of a compound according to any one of claims 7 - 12 for the preparation of a pharmaceutical composition for the treatment of diseases which are cured or relieved by the inhibition of kinases and/or phosphatases.

14. The use of a compound according to any one of claims 7 - 12 for the preparation of a pharmaceutical composition for the treatment of diseases like cell proliferation disorders, cardiovascular disorders, immunological diseases, inflammatory diseases, neuroimmunological diseases or autoimmune diseases.

15. The use of a compound according to any one of claims 7 - 12 and/or pharmaceutically acceptable salts thereof for the preparation of a pharmaceutical composition for prophylaxis and/or treatment of infectious diseases, including opportunistic infections.

16. The use according to claim 13 wherein the kinase is a herpesviral kinase.

17. The use according to claim 16 wherein the herpesviral kinase is UL97.

18. The use according to claim 15 wherein said infectious disease is herpes.

19. The use according to any one of claims 15 - 18 wherein said infectious disease is caused by herpes viruses.

20. The use according to claim 19 wherein the herpes virus is selected from the group comprising herpes simplex viruses, varicello viruses, cytomegalo viruses, muromegalo viruses, roseolo viruses, lymphocrypto viruses, and rhadino viruses.

21. The use according to any one of claims 13 - 20 wherein the compound of the general formula (I) or the compound of the general formula (la) and/or pharmaceutically acceptable salts thereof is administered in a dosage corresponding to an effective concentration in the range of 0.01 - 50 µM.

22. A pharmaceutical composition comprising at least one compound as defined in any one of claims 1 - 12 as an active ingredient and at least one pharmaceutically acceptable carrier, excipient and/or diluent.

23. A method for preventing and/or treating diseases which are cured or relieved by the inhibition of kinases and/or phosphatases in a mammal, including a human, which method comprises administering to the mammal an amount of at least one compound as defined in any one of claims 7 to 12 and/or pharmaceutically acceptable salts thereof, effective to prevent and/or treat said diseases which are cured or relieved by the inhibition of kinases and/or phosphatases.

24. A method for preventing and/or treating diseases like cell proliferation disorders, cardiovascular disorders, immunological diseases, inflammatory diseases, neuroimmunological diseases, autoimmune diseases in a mammal, including a human, which method comprises administering to the mammal an amount of at least one compound as defined in any one of claims 7 to 12 and/or pharmaceutically acceptable salts thereof, effective to prevent and/or treat said disease.

25. A method for preventing and/or treating an infectious disease, including opportunistic infections in a mammal, including a human, which method comprises administering to the mammal an amount of at least one compound as defined in any one of claims 7 to 12 and/or pharmaceutically acceptable salts thereof, effective to prevent and/or treat said infectious disease and/or opportunistic infection.

26. The method according to claim 23 wherein the kinase is a herpesviral kinase.

27. The method according to claim 26 wherein the herpesviral kinase is UL97.

28. The method according to claim 25 wherein said infectious disease is herpes.

29. The method according to any one of claims 23 - 28 wherein said infectious disease is caused by herpes viruses.

30. The method according to claim 29 wherein the herpes virus is selected from the group comprising herpes simplex viruses, varicello viruses, cytomegalo viruses, muromegalo viruses, roseolo viruses, lymphocrypto viruses, and rhadino viruses.

31. Method for preventing and/or treating herpesviral infections and/or associated diseases in a mammal, including a human, by administering a pharmaceutically effective amount of at least one compound as defined in any one of claims 7- 12 to said mammal, wherein said compound inhibits at least partially the activity of the herpesviral kinase UL97.

32. Method for identifying compounds useful for treating and/or preventing herpesviral infections and/or associated diseases comprising:
a) contacting a test compound with the herpesviral kinase UL97; and
b) determining a change in activity of said cellular kinase.

33. Method according to claim 32 wherein the test compound is a compound of the general formula (I) or a compound of the general formula (la) as defined in any one of claims 7-12.

34. Method for detecting herpesviral infections and/or associated diseases in an individual comprising:
a) providing a sample from said individual; and
b) determining activity in said sample of the herpesviral kinase UL97.

35. Method for detecting herpesviral infections and/or associated diseases in cells, cell cultures and/or cell lysates comprising:
a) providing a sample from said cells and/or cell lysates; and
b) determining activity in said sample of the herpesviral kinase UL97.

36. The method according to any one of claims 23 - 35 wherein the compound of the general formula (I) or the compound of the general formula (la) and/or pharmaceutically acceptable salts thereof is administered in a dosage corresponding to an effective concentration in the range of 0.01 - 50 µM.
